(19) Europäisches Patentamt
European Patent Office
Office européen des brevets

(11) Veröffentlichungsnummer: **0 019 745**
**B1**

(12) # EUROPÄISCHE PATENTSCHRIFT

(45) Veröffentlichungstag der Patentschrift: **28.07.82**

(21) Anmeldenummer: **80102417.5**

(22) Anmeldetag: **05.05.80**

(51) Int. Cl.³: **A 01 N 53/00**, A 01 N 37/22, A 01 N 43/00, A 01 N 47/08, C 07 C 103/30, C 07 C 125/065, C 07 C 125/067, C 07 C 127/19, C 07 C 155/02, C 07 D 521/00

(54) **2-Aminopropanalacetale, deren Herstellung, sie enthaltende Fungizide, deren Herstellung und Verfahren zur Bekämpfung von Pilzen.**

(30) Priorität: **19.05.79 DE 2920435**

(43) Veröffentlichungstag der Anmeldung: **10.12.80 Patentblatt 80/25**

(45) Bekanntmachung des Hinweises auf die Patenterteilung: **28.07.82 Patentblatt 82/30**

(84) Benannte Vertragsstaaten: **AT BE CH DE FR GB IT LI LU NL SE**

(56) Entgegenhaltungen:
**AT-B-318 295**
**AT-B-325 889**
**CH-A-591 805**
**CH-A-597 757**
**CH-A-606 028**
**CH-A-606 029**
**DE-A1-2 724 786**
**DE-A1-2 903 612**
**US-A-4 147 792**

(73) Patentinhaber: **BASF Aktiengesellschaft, Carl-Bosch-Strasse 38, D-6700 Ludwigshafen (DE)**

(72) Erfinder: **Ammermann, Eberhard, Dr., Sachsenstrasse 3, D-6700 Ludwigshafen (DE)**
Erfinder: **Zeeh, Bernd, Dr., Thorwaldsenstrasse 5, D-6700 Ludwigshafen (DE)**
Erfinder: **Thym, Sabine, Dr., Hasenhain 20, D-6900 Heidelberg-Dossenheim (DE)**
Erfinder: **Plath, Peter, Dr., Berner Weg 24, D-6700 Ludwigshafen (DE)**
Erfinder: **Rentzea, Costin, Dr., Neuenheimer Landstrasse 72, D-6900 Heidelberg (DE)**
Erfinder: **Eicken, Karl, Dr., Waldstrasse 63, D-6706 Wachenheim (DE)**
Erfinder: **Goetz, Norbert, Dr., Schoefferstrasse 25, D-6520 Worms (DE)**
Erfinder: **Pommer, Ernst-Heinrich, Dr., Berliner Platz 7, D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# 2-Aminopropanalacetale, deren Herstellung, sie enthaltende Fungizide, deren Herstellung und Verfahren zur Bekämpfung von Pilzen

Die vorliegende Erfindung betrifft neue 2-(N-Aryl-, N-acyl)-aminopropanalacetale, Verfahren zu ihrer Herstellung, ihre Anwendung als Fungizide und Verfahren zur Herstellung von substituierten Anilinen.

Es ist bekannt, dass N-Trichlormethylthiotetrahydrophthalimid ein gutes Fungizid zur Bekämpfung von pilzlichen Pflanzenkrankheiten, insbesondere zur Bekämpfung des falschen Mehltaus der Reben ist. (Chemical Week 1972, June 21, S. 63). Seine Wirkung ist jedoch gegen andere Phycomyceten, z.B. Phytophthora infestans bei Tomaten oder Kartoffeln nicht befriedigend. Es ist ferner bekannt, dass substituierte Chloracetanilide, die eine 1,3-Dioxolan-2-yl-methlygruppe tragen, eine herbizide Wirkung haben (DE-OS 2405510).

Es wurde nun gefunden, dass neue 2-(N-Aryl-, N-acyl)-aminopropanalacetale der Formel I

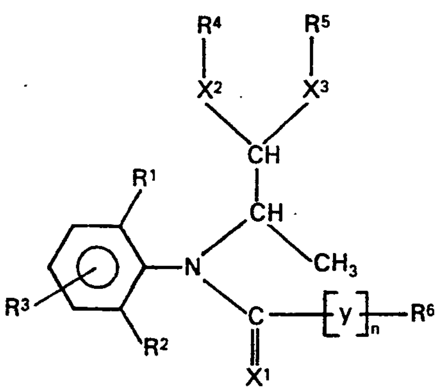

I

worin

$X^1$, $X^2$ und $X^3$ unabhängig voneinander Sauerstoff oder Schwefel,
Y Sauerstoff, Schwefel oder $NR^7$,
n 0 oder 1,
$R^1$ $C_1$-$C_4$-Alkyl,
$R^2$ Wasserstoff, Halogen, $C_1$-$C_4$-Alkyl oder $C_1$-$C_4$-Alkoxy,
$R^3$ Wasserstoff, Halogen oder $C_1$-$C_4$-Alkyl,
$R^4$ und $R^5$ unabhängig voneinander gegebenenfalls substituierte Alkyl- oder Arylalkylreste bedeuten oder zusammen als Alkylengruppe Teil eines gegebenenfalls durch Alkyl oder Aryl substituierten 5- oder 6-gliedrigen heterocyclischen Ringes sind,
$R^6$ einen gegebenenfalls substituierten Alkyl-, Alkenyl-, Cycloalkyl-, Cycloalkenyl- oder Heteroarylrest mit Ausnahme des Chlormethylrestes und des Acetylmethylrestes bedeutet und für den Fall, dass n = 1 ist, $R^6$ zusätzlich zu den oben genannten Bedeutungen einen gegebenenfalls substituierten Alkinyl-, Aryl-, Arylalkyl- oder Heteroarylalkylrest bedeutet und
$R^7$ Wasserstoff, eine Alkyl- oder Alkoxygruppe bedeutet, starke fungizide Eigenschaften aufweisen.

In der Formel I steht $R^1$ für einen $C_1$-$C_4$-Alkylrest, beispielsweise Methyl, Ethyl, n-Propyl, iso-Propyl oder Butyl. $R^2$ steht für Wasserstoff, für Halogen, z.B. Fluor, Chlor oder Brom, für eine $C_1$-$C_4$-Alkylgruppe, z.B. Methyl, Ethyl, Propyl oder Butyl, oder für einen $C_1$-$C_4$-Alkoxyrest, beispielsweise Methoxy, Ethoxy, Propoxy oder Butoxy. $R^3$ steht für Wasserstoff, Halogen, z.B. Fluor, Chlor oder Brom, oder für einen $C_1$-$C_4$-Alkylrest beispielsweise, Methyl, Ethyl, n-Propyl, iso-Propyl, n-Butyl oder tert.-Butyl. $R^4$ und $R^5$ stehen vorzugsweise für gegebenenfalls substituierte unverzweigte oder verzweigte Alkylreste mit bis zu 4 Kohlenstoffatomen, beispielsweise Methyl, Ethyl, n-Propyl, iso-Propyl, n-Butyl oder iso-Butyl, oder für gegebenenfalls substituierte Arylalkylreste, beispielsweise Benzyl oder 2-Phenylethyl-1.

$R^4$ und $R^5$ stehen insbesondere auch für eine Alkylengruppe mit 2 oder 3 Kohlenstoffatomen, die gegebenenfalls durch Aryl, beispielsweise Phenyl, oder durch Alkyl mit bis zu 4 Kohlenstoffatomen, beispielsweise Methyl, Ethyl oder Propyl, substituiert sein können.

$R^6$ steht beispielsweise für einen gegebenenfalls substituierten Alkylrest, der beispielsweise unverzweigt ist und 1 bis 20 Kohlenstoffatome enthält, z.B. Methyl, Ethyl, n-Butyl, n-Decyl oder n-Heptadecyl, oder verzweigt ist und 3 bis 15 Kohlenstoffatome enthält, beispielsweise Isopropyl, tert.-Butyl, 2-Methylbutyl-1 oder 2-Ethylhexyl-1. $R^6$ steht beispielsweise auch für einen geradkettigen oder verzweigten Alkenylrest mit 2 bis 15 Kohlenstoffatomen beispielsweise Vinyl, Propen-1-yl, 2-Methylpropen-1-yl, Hexen-1-yl oder Undecen-3-yl oder beispielsweise für einen cyclischen Alkyl- oder Alkenylrest mit 3 bis 10 Ringkohlenstoffatomen beispielsweise für Cyclopropyl, Cyclobutyl, Cyclohexyl oder Cyclohexen-2-yl, ferner beispielsweise für einen gegebenenfalls substituierten Heteroarylrest mit 5 bis 6 Ringatomen und 1 bis 3, insbesondere 1 bis 2 Heteroatomen im Ring, die gleich oder verschieden sein können und vorzugsweise Sauerstoff, Stickstoff oder Schwefel sind, wie beispielsweise in einem Furan-, Thiophen-, Isoxazol- oder Pyridinrest. Ausserdem steht $R^6$ bevorzugt für einen gegebenenfalls substituierten Azolylalkylrest mit 1 bis 2 Kohlenstoffatomen im Alkylteil und beispielsweise Pyrazolyl, Imidazolyl oder Triazolyl als Azolylrest. Für den Fall, dass n = 1 ist, steht $R^6$ zusätzlich auch für einen gegebenenfalls substituierten Alkinylrest mit 3 bis 6 Kohlenstoffatomen beispielsweise für Propin-2-yl-1 oder 1,1-Dimethyl-propin-2-yl-1.

Ausserdem steht dann $R^6$ bevorzugt für einen gegebenenfalls substituierten Arylrest wie beispielsweise für einen Phenyl- oder Naphthylrest oder für einen gegebenenfalls substituierten Arylalkyl- oder Heteroarylalkylrest mit 1 bis 2 Kohlenstoffatomen im Arylteil bzw. mit 5 bis 6 Ringatomen im Heteroarylteil, von denen 1 bis 3,

insbesondere 1 und 2, Heteroatome wie Sauerstoff, Stickstoff oder Schwefel sein können, wie beispielsweise in einem Thiophen-, Isoxazol- oder Pyridinring.

Als bevorzugte Substituenten der oben erläuterten für $R^6$ stehenden Reste seien beispielsweise genannt: Halogen, insbesondere Fluor, Chlor oder Brom; Nitro; ein geradkettiger Alkylrest mit 1 bis 4 Kohlenstoffatomen; ein verzweigter oder cyclischer Alkylrest mit 3 bis 6 Kohlenstoffatomen; ein Alkoxy-, Alkoxyalkoxy- oder Alkylthiorest mit 1 bis 8 Kohlenstoffatomen, beispielsweise eine Methoxy-, Äthoxy- oder Isopropoxygruppe oder eine Methylthio-, Äthylthio- oder n-Butylthiogruppe; ein Halogenalkyl-, Halogenalkoxy-, Halogenalkylthio- oder Halogenalkylsulfonylrest mit bis zu 4 Kohlenstoffatomen und bis zu 9 Halogenatomen, insbesondere Fluor oder Chlor, beispielsweise ein Trichlormethyl, Trifluormethyl, oder Tetrafluoräthoxyrest; eine Formyl-, Cyano- oder Thiocyanatogruppe; ein Alkoxycarbonyl-, Alkoxysulfonyl-, Alkylcarbonyl-, Alkylcarbamoyl-, Amino-, Acylamino- oder ein Alkylaminosulfonylrest mit 2 bis 10 Kohlenstoffatomen, beispielsweise ein Methoxycarbonyl-, Acetyl-, Propionyl-, N,N-Dimethylcarbamoyl- oder ein Butyrylaminorest; ein Alkylamino-, Dialkylamino- oder ein Azolylrest, beispielsweise ein Methylamino-, Diäthylamino-, Pyrazolyl-, Imidazolyl- oder ein Triazolylrest; ein gegebenenfalls substituierter Phenyl- oder Phenylcarbonylrest mit 6 bis 11 Kohlenstoffatomen; ein gegebenenfalls insbesondere in der 4-Stellung substituierter Aryloxy- oder Arylalkyloxyrest, beispielsweise ein 4-Chlorphenoxy- oder ein 2,4-Dichlorphenoxyrest; ein gegebenenfalls substituierter Arylthio, Arylsulfinyl- oder ein Arylsulfonylrest. $R^7$ steht für Wasserstoff oder einen Alkyl- oder Alkoxyrest mit 1 bis 4 Kohlenstoffatomen.

Die neuen 2-(N-Aryl-, N-acyl-)amino-propanalacetale besitzen im Kohlenstoffatom 2 des Propanals ein Asymmetriezentrum und je nach der Beschaffenheit von $R^6$ weitere Asymmetriezentren. Mit üblichen Methoden können die optisch reinen Enantiomeren bzw. die Diastereomeren erhalten werden. Die vorliegende Erfindung umfasst auch diese Verbindungen. Als Fungizide kann man sowohl die reinen Enantiomeren bzw. die einheitlichen Diastereomeren verwenden wie auch die bei der Synthese üblicherweise anfallenden Gemische.

Die für die Herstellung der neuen Verbindungen benötigten Aniline der Formel II,

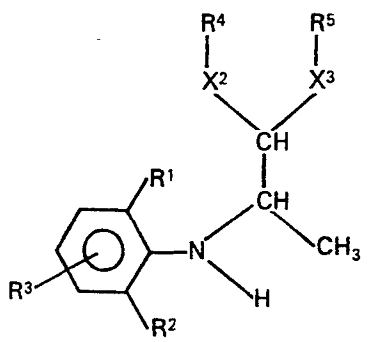 II

in der $R^1$, $R^2$, $R^3$, $R^4$, $R^5$, $X^2$ und $X^3$ die oben erläuterten Bedeutungen haben, sind teilweise bekannt. So wurde von M. Chastrette (Ann. Chim. [Paris] 7, 643-668 [1962]) die Synthese von 2-(N-2'-Methylphenyl)-aminopropanaldiethylacetal beschrieben (ibd. S. 654, Tabelle II, 4. Zeile) durch Umsetzung von 2-Brompropanaldiethylacetal mit 2-Methylanilin (ibd. S. 656, 657; Ausbeute 24%) oder durch Reaktion von Methylmagnesiumjodid mit N-Phenyliminoglyoxyldiethylacetal (ibd. S. 658; Ausbeute 72%).

Ferner ist ein Teil der als Ausgangsstoffe benutzten Aniline der Formel II Gegenstand der DE-OS 2802211. Dort wird ein Anilin mit cyclischer Acetalstruktur beschrieben, und zwar das 2-(N-2-Methyl-6-ethylphenyl)-aminopropanalethylenglykolacetal.

Es wurde nun gefunden, dass man die Aniline der Formel XII

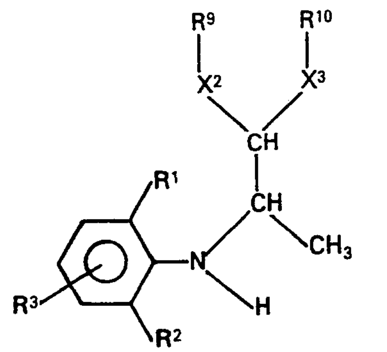 XII

worin $R^1$, $R^2$, $R^3$, $X^2$ und $X^3$ die oben erläuterten Bedeutungen haben und $R^9$ und $R^{10}$ unabhängig voneinander für gegebenenfalls substituierte Alkyl- oder Arylalkylreste stehen, vorteilhaft erhält, wenn man ein Methylglyoxalacetal der Formel XIII

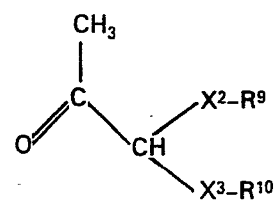 XIII

mit einem Anilin der Formel XIV

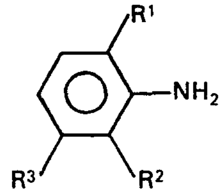 XIV

umsetzt und die als Umsetzungsprodukt erhaltene Schiff'sche Base hydriert, z.B. mit komplexen Hydriden oder auf katalytischem Wege.

In der Formel XII stehen $R^9$ und $R^{10}$ vorzugsweise für gegebenenfalls substituierte unverzweigte oder verzweigte Alkylreste mit bis zu 4 Kohlenstoffatomen, beispielsweise Methyl, Ethyl, n-Propyl, iso-Propyl, n-Butyl oder iso-Butyl, oder für

gegebenenfalls substituierte Arylalkylreste, beispielsweise den Benzyl- oder den 2-Phenylethyl-1-Rest.

Bei dem erfindungsgemässen Verfahren wird die Entstehung anorganischer Salze vermieden, die als Nebenprodukte in grossen Mengen bei der bekannten Umsetzung von α-Halogenpropanalacetalen mit Anilinen durch Umsetzung des entstehenden Halogenwasserstoffs mit anorganischen Hilfsbasen entstehen. Ausserdem ist das neue Verfahren einfacher durchzuführen, da auf absolut trockene Reaktionsbedingungen, wie sie die bekannte Grignardreaktion erfordert, verzichtet werden kann. Daher ist das erfindungsgemässe Verfahren umweltfreundlicher und einfacher als die bekannten Verfahren.

Ein weiterer Vorteil des neuen Verfahrens ist, dass es nach folgender Arbeitsweise möglich ist, die Aniline der Formel XII in grösserem Massstab als im Labormassstab herzustellen:

1. Herstellung der Schiffschen Base der allgemeinen Formel XV

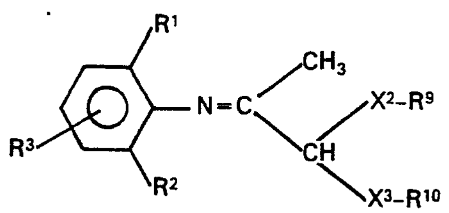

XV

durch Erhitzen der Reaktionsmischung am Rückfluss und Wasserauskreisung und

2. anschliessende Hydrierung.

Der erste Reaktionsschritt – die Herstellung der Schiff'schen Base – wird beispielsweise so durchgeführt, dass man 1 Mol des Anilins der Formel XIV mit 0,9 bis 1,5 Mol des Methylglyoxalacetals der Formel XIII in einem Lösungsmittel gegebenenfalls unter Zusatz eines Katalysators umsetzt und die Reaktion bei einer Temperatur von 40 bis 200°C, vorzugsweise von 50 bis 120°C, drucklos oder unter Druck, kontinuierlich oder diskontinuierlich durchführt. Zweckmässig verwendet man unter den Reaktionsbedingungen inerte Lösungsmittel. Als Lösungsmittel kommen z.B. in Frage:

Aromatische Kohlenwasserstoffe, z.B. Benzol, Toluol, Äthylbenzol, o-, m-, p-Xylol, Isopropylbenzol, Methylnaphthalin; aliphatische oder cycloaliphatische Kohlenwasserstoffe, z.B. Heptan, Pinan, Nonan, Benzinfraktionen innerhalb eines Siedepunktintervalls von 70 bis 190°C, Cyclohexan, Methylcyclohexan, Dekalin, Hexan, Ligroin, 2,2,4-Trimethylpentan, 2,2,3-Trimethylpentan, 2,3,3-Trimethylpentan, Octan; und entsprechende Gemische. Zweckmässig verwendet man das Lösungsmittel in einer Menge von 150 bis 10 000 Gewichtsprozent, vorzugsweise von 300 bis 600 Gewichtsprozent, bezogen auf Ausgangsstoff XIV.

Die Umsetzung kann wie folgt durchgeführt werden: Ein Gemisch der Ausgangsstoffe XIII, XIV und das Lösungsmittel werden während 2 bis 15 Stunden bei der Reaktionstemperatur gehalten. Vorteilhaft kann man das entstehende Wasser noch während der Reaktion kontinuierlich, z.B. durch azeotrope Destillation, mit einem geeigneten Lösungsmittel, wie Cyclohexan, entfernen. Dann kann die Schiff'sche Base aus dem Gemisch in üblicher Weise, z.B. durch fraktionierte Destillation, abgetrennt und mit der isolierten, gereinigten Schiff'schen Base der zweite Schritt der Umsetzung durchgeführt werden. Diese Arbeitsweise wird man aber in der Regel schon aus wirtschaftlichen Gründen nicht wählen, sondern vorteilhaft nach der Herstellung der Schiff'schen Base das Lösungsmittel abdestillieren, ein für die Hydrierung geeignetes Lösungsmittel zusetzen und die Hydrierung bei der Reaktionstemperatur durchführen.

Für die Hydrierung eignen sich sowohl die Reduktion mit komplexen Hydriden wie $NaBH_4$ oder $LiAlH_4$ als auch die katalytische Hydrierung.

Die Reduktion mit Natriumborhydrid wird im allgemeinen so durchgeführt, dass man in einem Lösungsmittel die Schiff'schen Basen mit 0,2 Mol bis 1 Mol, vorzugsweise 0,25 bis 0,5 Mol, Natriumborhydrid pro Mol Schiff'sche Base bei einer Temperatur zwischen −20 und +20°C drucklos oder unter Druck, kontinuierlich oder diskontinuierlich umsetzt.

Bei der katalytischen Hydrierung werden dem Reaktionsgemisch am Anfang und im Verlauf der Umsetzung solche Mengen an Wasserstoff zugeführt, dass sich bei der Umsetzungstemperatur stets ein entsprechender Reaktionsdruck, zweckmässig zwischen 150 und 300 bar, einstellt. Die Umsetzung wird im allgemeinen bei einer Temperatur von 20 bis 200°C, vorzugsweise von 25 bis 160°C, diskontinuierlich oder kontinuierlich durchgeführt. Zur entsprechenden Druckeinstellung können auch inerte Gase wie Stickstoff verwendet werden.

Geeignete Lösungsmittel für beide Verfahrensvarianten sind solche, die unter den Reaktionsbedindungen inert sind, wie Alkanole und Cycloalkanole z.B. n-Butanol, Isobutanol, tert.-Butanol, Glykol, Glycerin, n-Propanol, Isopropanol, Amylalkohol, Cyclohexanol, 2-Methyl-4-pentanol, Äthylenglykolmonoäthyläther, 2-Äthylhexanol, Methylglykol und insbesondere Äthanol, Methanol; cyclische Äther wie Tetrahydrofuran oder Dioxan. Es kommen auch die schon für den ersten Reaktionsschritt genannten Lösungsmittel in Betracht. Bevorzugt werden die für den ersten Reaktionsschritt genannten Lösungsmittelmengen verwendet.

Zur katalytischen Hydrierung wird der Katalysator in der Regel in einer Menge von 5 bis 30 Gewichtsprozent, bezogen auf die Schiff'sche Base, verwendet. Er kann im Gemisch mit einem für die Umsetzung geeigneten Trägermaterial, z.B. Siliciumdioxid, zur Anwendung gelangen, wobei zweckmässig die Menge des Katalysators 10 bis 40 Gewichtsprozent des Gemisches von Katalysator und Träger beträgt.

Zweckmässig verwendet man Kupferchromitkatalysatoren, z.B. entsprechende Kupfer-Chromoxidkatalysatoren wie die von H. Adkins verwendeten Kupferchromite. Sie enthalten beispielsweise Kupfer-Chrom-Spinell ($CuCr_2O_4$) oder Gemische im Verhältnis 5 CuO : 4 $Cr_2O_3$ bzw. gehen von solchen Verbindungen aus und können noch andere Oxide, hauptsächlich die der Erdalkalimetalle wie Barium, Calcium oder Magnesium, enthalten.

Bezüglich der Herstellung von Kupferchromitkatalysatoren wird auf Houben-Weyl, Methoden der Organischen Chemie, Band 4/2, Seiten 180 bis 183 und Journal of Applied Chemistry, Band 5 (1955), Seiten 289 bis 295, verwiesen.

Folgende Verfahrensbeschreibung erläutert die Herstellung von Anilinen der Formel XII:

1. Reaktionsschritt

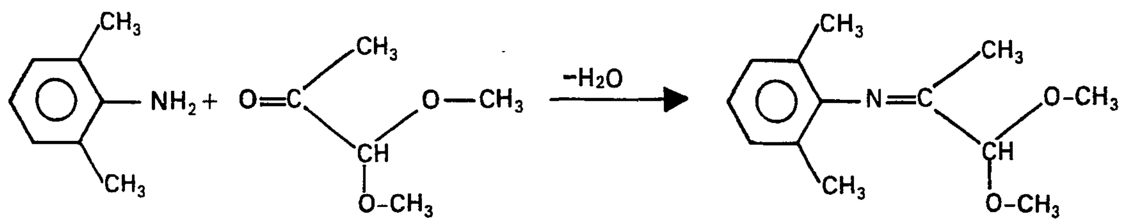


121 Teile (Gewichtsteile) 2,6-Dimethylanilin, 118 Teile Methylglyoxaldimethylacetal und 0,2 Teile p-Toluolsulfonsäure werden in 500 Teilen Cyclohexan für 4 Stunden am Rückfluss erhitzt bis 18 Teile Wasser azeotrop abdestilliert und aus dem Destillat abgetrennt sind. Dann wird das Lösungsmittel abdestilliert und der Rückstand direkt weiter umgesetzt.
Ausbeute: 212 Teile (96%) Schiff'sche Base.

2. Reaktionsschritt

a) Katalytische Hydrierung

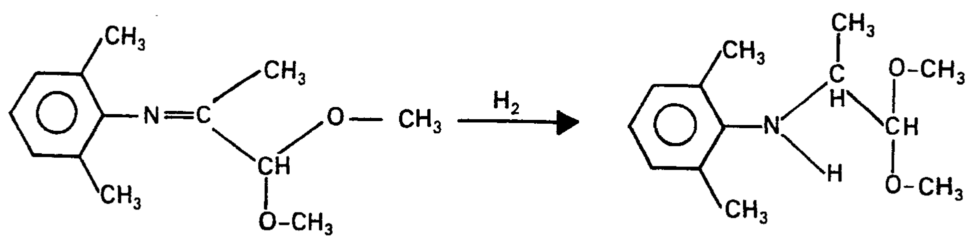


Ein Hydrierautoklav mit einem Volumen von 1000 Raumteilen wird mit 200 Teilen der Schiff'schen Base aus 2,6-Dimethylanilin und Methylglyoxaldimethylacetal, die in 500 Teilen Tetrahydrofuran gelöst sind, und 15 Teilen Adkins-Katalysator (Kupferchromit in pulverisierter Form) gefüllt.

Anschliessend wird der Autoklav auf 160°C erhitzt und Wasserstoff bis zum Erreichen eines Druckes von 200 bar aufgepresst. Sobald die Wasserstoffaufnahme beendet und ein konstanter Druck erreicht ist (nach ca. 7 Stunden), wird abgekühlt und aufgearbeitet.

Der Katalysator wird vom Reaktionsgemisch durch Filtration abgetrennt. Aus dem Filtrat werden durch destillative Reinigung 129 Teile 2-(N-2',6'-Dimethylphenyl)-aminopropanal-dimethylacetal, $Kp_{0,4}$ = 90 bis 91°C, erhalten.
Die Ausbeute beträgt 64% d.Th.

b) Reduktion mit $NaBH_4$
220 Teile der Schiff'schen Base aus 2,6-Dimethylanilin und Methylglyoxaldimethylacetal werden in 1000 Teilen Methanol gelöst und bei 0°C portionsweise mit 57 Teilen Natriumborhydrid versetzt. Nach dem Rühren über Nacht wird das Lösungsmittel abdestilliert, der Rückstand in 1000 Teilen Wasser gelöst und die Lösung viermal mit 300 Teilen Methylenchlorid extrahiert. Nach dem Abdestillieren des Methylenchlorids verbleiben als Rückstand 205 Teile Rohprodukt, das bei 81°C/0,1 mm destilliert wird.
Ausbeute: 182 Teile (81%).

Analog können folgende Aniline der Formel XII hergestellt werden:

Tabelle I

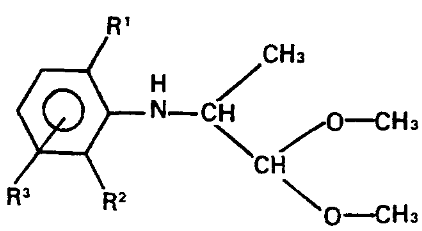

| $R^1$ | $R^2$ | $R^3$ | Kp °C mbar |
|---|---|---|---|
| $CH_3$ | H | H | 75/0,1 |
| $C_2H_5$ | H | H | 84/0,1 |
| $CH_3$ | $C_2H_5$ | H | 86–88/0,1 |
| $CH_3$ | H | 5-tert-$C_4H_9$ | 100–102/0,1 |
| $C_2H_5$ | $C_2H_5$ | H | 92–93/0,1 |
| $CH_3$ | Cl | H | 90–92/0,1 |
| $CH_3$ | $CH_3$ | 4-$CH_3$ | 115/0,3 |
| $CH_3$ | $CH_3$ | 3-$CH_3$ | 94/0,4 |

Die neuen Verbindungen der allgemeinen Formel I kann man herstellen, indem man ein Anilin der Formel II

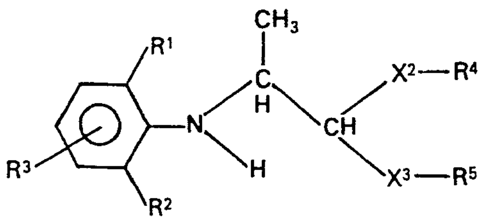

II

worin $R^1$, $R^2$, $R^3$, $R^4$, $R^5$, $X^2$ und $X^3$ die oben angegebenen Bedeutungen haben,

a) mit einem Säurehalogenid der Formel III

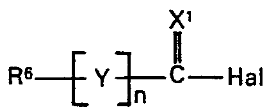

III

oder

b) mit einem Säureanhydrid der Formel IV

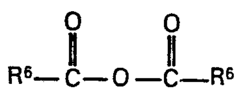

IV

oder

c) mit einem Isocyanat der Formel V

$R^6$-N=C=$X^1$ V

worin $X^1$, Y, n und $R^6$ die oben angegebenen Bedeutungen haben, gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers, bei Temperaturen zwischen 0 und 120°C umsetzt. Zu den bevorzugten Lösungs- bzw. Verdünnungsmitteln gehören Halogenkohlenwasserstoffe, beispielsweise Methylenchlorid, Chloroform, 1,2-Dichloräthan, Chlorbenzol; aliphatische oder aromatische Kohlenwasserstoffe wie Cyclohexan, Petroläther, Benzol, Toluol oder Xylole; Ester wie Essigsäureäthylester; Nitrile wie Acetonitril; Sulfoxide, wie Dimethylsulfoxid; Ketone wie Aceton oder Methyläthylketon; Äther wie Diäthyläther, Tetrahydrofuran oder Dioxan oder entsprechende Gemische.

Zweckmässig verwendet man das Lösungs- bzw. Verdünnungsmittel in einer Menge von 100 bis 2000 Gew.%, vorzugsweise 100 bis 1000 Gew.%, bezogen auf die Einsatzstoffe II bzw. III, IV oder V.

Geeignete anorganische oder organische Basen, die gegebenenfalls auch als säurebindende Mittel bei der Reaktion verwendet werden können, sind beispielsweise Alkalicarbonate wie Kalium- oder Natriumcarbonat; Alkalihydride wie Natriumhydrid oder tertiäre Amine wie Trimethylamin, Triäthylamin, N,N-Dimethylanilin, N,N-Dimethylcyclohexylamin, N-Methylpiperidin oder Pyridin; Azole wie 1,2,4-Triazol oder Imidazol. Es können aber auch andere übliche Basen verwendet werden.

Als Reaktionsbeschleuniger kommen vorzugsweise Metallhalogenide wie Natriumbromid oder Kaliumjodid, Azole wie Imidazol oder 1,2,4-Triazol oder Pyridine wie 4-Dimethylaminopyridin oder Kombinationen dieser Reaktionsbeschleuniger in Frage.

Die Umsetzung wird im allgemeinen bei Temperaturen zwischen 0 und 120°C in einem Zeitraum von 1 bis 60 Stunden durchgeführt, drucklos oder unter Druck, kontinuierlich oder diskontinuierlich.

Man verfährt im allgemeinen so, dass man auf 1 Mol der Verbindung II jeweils 0,9 bis 1,3 Mol der Verbindungen III bzw. IV bzw. V sowie 0,5 bis 2 Mol Base und gegebenenfalls 0,01 Mol bis 0,1 Mol eines Reaktionsbeschleunigers einsetzt.

In einer bevorzugten Form des erfindungsgemässen Verfahrens vermischt man den Ausgangsstoff II gegebenenfalls mit einer Base und gegebenenfalls mit einem Verdünnungsmittel, gibt dann den Einsatzstoff III bzw. IV, bzw. V und gegebenenfalls einen Reaktionsbeschleuniger zu und hält das Reaktionsgemisch für 0,5 bis 12, vorzugsweise 1 bis 6 Stunden, bei der Reaktionstemperatur, die zwischen 0 und 120°C liegen kann.

Zur Isolierung der neuen Verbindungen wird gegebenenfalls das Verdünnungsmittel entfernt, der Rückstand in einem geeigneten Lösungsmittel gelöst und mit wässriger, verdünnter Säure, dann mit wässriger verdünnter Lauge sowie mit Wasser gewaschen, um die überschüssige Base und die Ausgangsstoffe II, III, IV und V zu entfernen.

Die nach dem Abdestillieren des Lösungsmittels verbleibenden Produkte bedürfen im allgemeinen keiner weiteren Reinigung, können aber nötigenfalls nach bekannten Methoden wie Umkristallisation, Extraktion oder Chromatographie weiter gereinigt werden.

Es wurde weiterhin gefunden, dass man eine Teilmenge der erfindungsgemässen Verbindungen der Formel I herstellen kann, indem man ein Anilin der Formel II

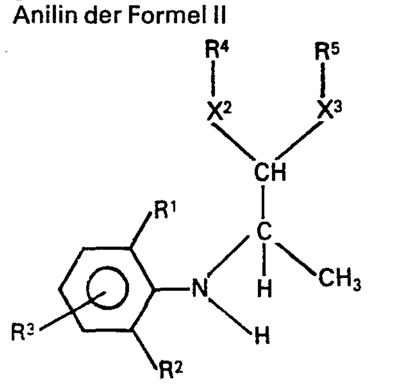

II

worin $R^1$, $R^2$, $R^3$, $R^4$, $R^5$, $X^2$ und $X^3$ die oben erläuterten Bedeutungen haben, zunächst

a) mit einem Phosgen der Formel VI

$X^1 = CCl_2$ VI

worin $X^1$ die oben angegebene Bedeutung hat, zu einer Verbindung der Formel VII umsetzt

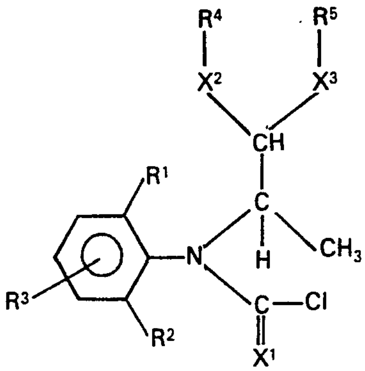

VII

oder

b) mit einer Verbindung der Formel VIII

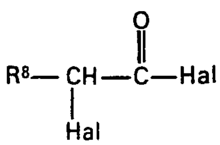

VIII

worin $R^8$ für Wasserstoff steht oder die für $R^6$ angegebenen Bedeutungen hat und Hal ein Halogenatom bedeutet, zu einer Verbindung der Formel IX umsetzt

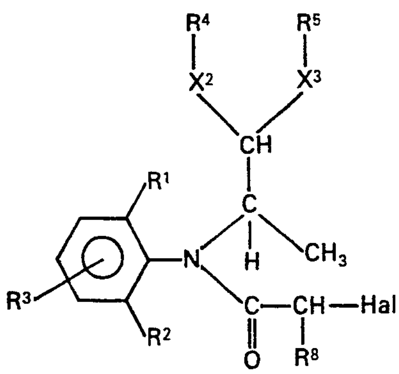

IX

und dann die Verbindungen der Formeln VII bzw. IX mit nucleophilen Verbindungen der Formel X

$R^6$- YH X

worin $R^6$ und Y die oben erläuterten Bedeutungen haben, gegebenenfalls in Gegenwart eines Lösungs- oder Verdünnungsmittels, gegebenenfalls in Gegenwart einer anorganischen oder organischen Base gegebenenfalls unter Zusatz eines Reaktionsbeschleunigers bei Temperaturen zwischen -20 und +100°C umsetzt.

Zu den bevorzugten Lösungs- bzw. Verdünnungsmitteln und zu den anorganischen oder organischen Basen gehören die oben erläuterten.

Die erfindungsgemässe Umsetzung wird beispielsweise bei Temperaturen zwischen -20 und +100°C durchgeführt, drucklos oder unter Druck, kontinuierlich oder diskontinuierlich.

Für die Isolierung der Endprodukte gelten die oben gegebenen Erläuterungen.

Eine weitere Untergruppe der erfindungsgemässen Verbindungen der allgemeinen Formel I erhält man, indem man Verbindungen der Formel XI

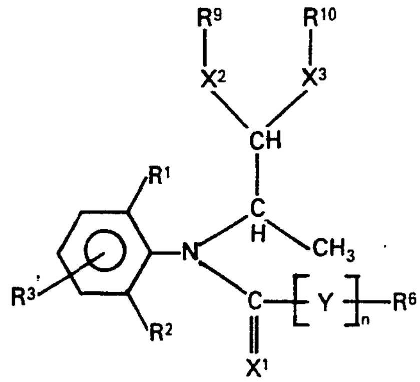

XI

worin $R^1$, $R^2$, $R^3$, $R^6$, $R^9$, $R^{10}$, $X^1$, $X^2$, $X^3$, Y und n die oben erläuterten Bedeutungen haben mit 1,2- oder 1,3-Diolen, -Dithiolen oder -Mercaptoalkoholen zu cyclischen Acetalen bei Temperaturen zwischen 0 und 120°C gegebenenfalls in Gegenwart eines Lösungs- oder Verdünnungsmittels und gegebenenfalls unter Zusatz eines Reaktionsbeschleunigers umacetalisiert.

Zu den oben genannten 1,2- oder 1,3-Diolen, -Dithiolen oder -Mercaptoalkoholen gehören beispielsweise Ethylenglykol, Mercaptoethanol, Ethandithiol-1,2, Propylenglykol-1,2, Propan-diol-1,3, Propandithiol-1,3, 2-Methyl-, 2-phenylpropandiol-1,3, 2-Methyl-2-mercaptoethanol-1, Butandiol-1,3, Butandiol-2,3 und Neopentylglykol.

Als Lösungs- oder Verdünnungsmittel eignen sich die oben genannten Flüssigkeiten oder die 1,2- oder 1,3-Diole, -Dithiole oder -Mercaptoalkohole bei Anwendung in stöchiometrischer Menge oder im Überschuss. Als Reaktionsbeschleuniger eignen sich Lewis- oder Protonsäuren, beispielsweise $ZnCl_2$, $AlCl_3$ oder $BF_3$ oder Mineralsäuren oder Sulfonsäuren. Für die Isolierung der Endprodukte gelten die obigen Erläuterungen.

Weitere Untergruppen der erfindungsgemässen Verbindungen der allgemeinen Formel I erhält man, wenn man je nach der funktionellen Beschaffenheit von $R^6$ übliche chemische Folgereaktionen vornimmt.

Die Herstellung der neuen Verbindungen wird durch folgende Beispiele erläutert:

Beispiel 1

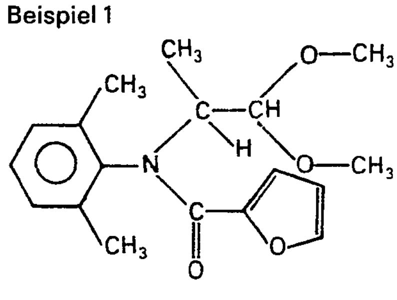

33,5 Teile (Gewichtsteile) 2-(N-2',6'-Dimethylphenyl)-aminopropanaldimethylacetal werden in 300 Teilen Toluol gelöst und mit 25 Teilen Triethylamin versetzt. Bei der Zugabe von 20 Teilen Furan-2-carbonsäurechlorid steigt die Temperatur auf 30°C. Anschliessend wird das Reaktionsgemisch für 2 Stunden bei 70°C gerührt. Nach dem Abkühlen wird das Reaktionsgemisch zweimal mit 100 Teilen 2N-Salzsäure, zweimal mit 100 Teilen 2N-Natronlauge und zweimal mit 100 Teilen Wasser gewaschen. Nach dem Trocknen wird das Lösungsmittel abdestilliert. Der verbleibende Rückstand wird aus n-Pentan umkristallisiert. Man erhält 28 Teile (59% d. Th.) 2-(N-2',6'-Dimethylphenyl-, N-2''-furan-carbonyl)-aminopropanaldimethylacetal vom Schmelzpunkt 75 bis 77°.

Beispiel 2

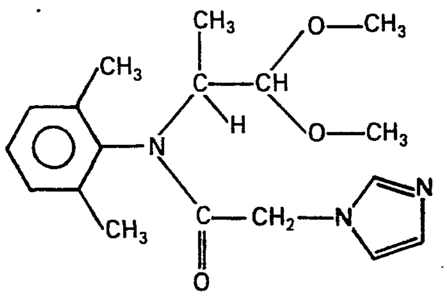

30 Teile 2-(N-2',6'-Dimethylphenyl-, N-chloracetyl)-amino-propanaldimethylacetal, 20,4 Teile Imidazol, 50 Teile Dioxan und 20 Teile Wasser werden für 8 Stunden auf Rückflusstemperatur erhitzt. Nach dem Eindampfen im Vakuum wird der Rückstand in 100 Teilen Methylenchlorid gelöst, dreimal mit je 50 Teilen Wasser gewaschen, über Natriumsulfat getrocknet und eingeengt. Nach dem Reinigen des Rohproduktes durch Filtration der Lösung in Essigester über Kieselgel isoliert man 18,1 Teile einheitliches Öl.

| | | | |
|---|---|---|---|
| Ber.: | C 65,2 | H 7,6 | N 12,6 |
| Gef.: | C 64,8 | H 7,8 | N 12,1. |

Beispiel 3

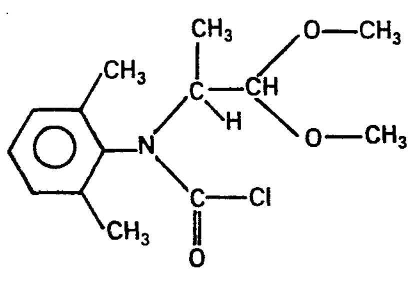

a) Zu einer Lösung von 45 Gewichtsteilen Phosgen in 200 Teilen Toluol wird bei -10°C eine Lösung von 85 Teilen 2(N-2',6'-Dimethylphenyl)-aminopropanal-dimethylacetal in 150 Teilen Toluol zugetropft.

Man rührt 4 Stunden nach, wobei die Temperatur auf Raumtemperatur ansteigt. Dann wird das Lösungsmittel abdestilliert. Es bleiben 120 Teile des N(2,6-Dimethylphenyl)-N-(1',1'-dimethoxy-2'-propyl) carbaminsäurechlorids als braunes Öl zurück, dessen UR-Spektrum keine NH-Bande mehr aufweist.

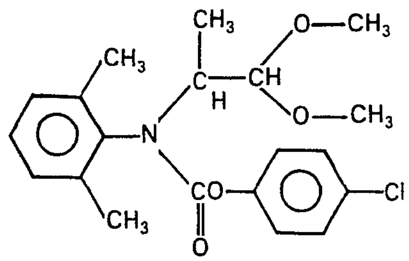

b) Zu einer Lösung von 30 Teilen Natrium-4-chlorphenolat in 200 Teilen Tetrahydrofuran wird eine Lösung von 54 Teilen N-(2,6-Dimethylphenyl)-N-(1',1'-dimethoxy-2'-propyl)-carbaminsäurechlorid in 100 Teilen Tetrahydrofuran getropft und über Nacht nachgerührt. Das Reaktionsgemisch wird eingeengt, in Methylenchlorid gelöst, zweimal mit Wasser gewaschen, getrocknet und eingeengt. Das verbleibende Öl wird an Kieselgel chromatographisch mit Cyclohexan mit steigendem Essigsäureethylester-Zusatz als Elutionsmittel gereinigt. Man erhält 10 Teile des O-(4-Chlorphenyl)-N-(2',6'-dimethylphenyl)-N-(1'',1''-dimethoxy-2''-propyl)-carbamats als analysenreines Öl;
$n_D^{20} = 1.5425$.

Beispiel 4

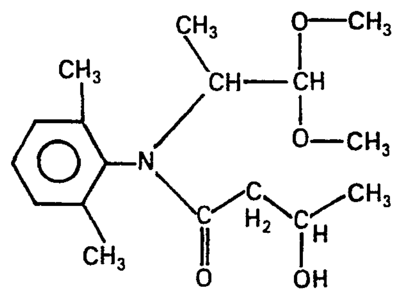

Zu einer Lösung von 61,4 Teilen 2,6-Dimethyl-N-(1',1'-dimethoxy-2'-propyl)-acetoacetanilid in 500 Teilen Methanol werden bei 0 bis +5°C portionsweise 7,6 Teile Natriumborhydrid gegeben. Nach dem Rühren über Nacht werden 40 Teile Eisessig zugesetzt und das Reaktionsgemisch zur Trockne eingeengt. Der Rückstand wird in 500 Teilen Diethylether und 400 Teilen 10%iger wässriger Natriumcarbonatlösung gelöst. Die organische Phase wird abgetrennt, getrocknet und eingeengt.

Man erhält 50,2 Teile (81% der Theorie) 2,6-Dimethyl-N-(1',1'-dimethoxy-2'-propyl)-3''-hydroxybutyranilid als analysenreines Öl.

Das als Ausgangsprodukt verwendete 2,6-Dimethyl-N-(1',1'-dimethoxy-2'-propyl)-acetoacetanilid wird wie folgt hergestellt:

Zu einer Lösung von 111,5 Teilen 2-(N-2',6'-Dimethylphenyl)-amino-propanaldimethylacetal in 250 Teilen Toloul werden 3 Teile Triethylamin und bei 80 - 90°C unter Rühren 39,5 Teile Diketen getropft. Anschliessend wird für 3 Stunden nachgerührt. Das abgekühlte Reaktionsgemisch wird einmal mit 2 normaler Salzsäure und zweimal mit Wasser gewaschen, getrocknet und eingeengt. Man erhält 121,8 Teile (78,8% der Theorie) 2,6-Dimethyl-N-(1',1'-dimethoxy-2'-propyl)-acetoacetanilid als analysenreines Öl.

Beispiel 5

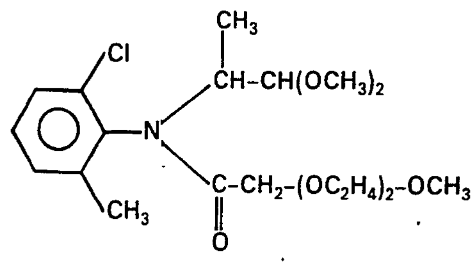

Zu einer Lösung von 36,5 Teilen 2-(N-2-Methyl-6-chlorphenyl)-aminopropanaldimethylacetal in 200 Teilen Methylenchlorid werden 16,5 Teile Triethylamin gegeben. Unter Rühren und Kühlung werden 29,4 Teile 3,6,9-Trioxadecansäurechlorid zugetropft und über Nacht nachgerührt. Der Niederschlag wird abgesaugt, die organische Phase dreimal mit Wasser gewaschen, getrocknet und eingeengt. Es verbleiben 30 Teile (2-Methyl-6-chlor-N-(1'-1'-dimethoxy-2'-propyl)-)-2,6,9-trioxadecansäureanilid als analysenreines Öl ($n_D^{20} = 1.5042$).

Analog werden folgende Verbindungen hergestellt:

Tabelle 2

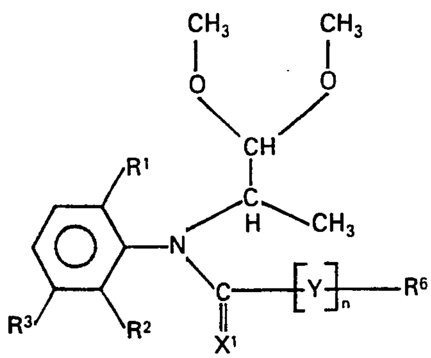

| Nr. | $R^1$ | $R^2$ | $R^3$ | $X^1$ | Y | n | $R^6$ | Kp/$n_D^{20}$ |
|---|---|---|---|---|---|---|---|---|
| 6 | $CH_3$ | Cl | H | O | – | O | $CH_2OCH_3$ | 1,5235 |
| 7 | $CH_3$ | H | 5-tert-$C_4H_9$ | O | – | O | $CH_2$-$OCH_3$ | Harz |
| 8 | $C_2H_5$ | $C_2H_5$ | 5-tert-$C_4H_9$ | O | – | O | $CH_2$-$OCH_3$ | 143/0,15 mbar |
| 9 | $CH_3$ | H | H | O | – | O | $CH_2$-$OCH_3$ | 1,5080 |
| 10 | $CH_3$ | $CH_3$ | H | O | – | O | $CH_2$-$CH_2$-O-$CH_3$ | 1,5000 |
| 11 | $CH_3$ | $CH_3$ | H | O | – | O | $CH_2$-CHOH-$C_2H_5$ | |
| 12 | $CH_3$ | $CH_3$ | H | O | – | O | $CH_2$-CH($OCOCH_3$)-$CH_3$ | Öl |
| 13 | $CH_3$ | $CH_3$ | H | O | – | O | 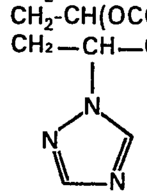 | |
| 14 | $CH_3$ | $CH_3$ | H | O | – | O | $CH_2$- $CH_3$ | 1,5055 |
| 15 | $C_2H_5$ | $C_2H_5$ | H | O | – | O | 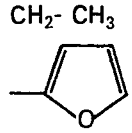 | 168–171/0,1 mbar |
| 16 | $CH_3$ | H | H | O | – | O | 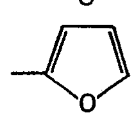 | Fp 88°C |

Tabelle 2 (Fortsetzung)

| Nr. | $R^1$ | $R^2$ | $R^3$ | $X^1$ | Y | n | $R^6$ | $Kp/n_D^{20}$ |
|---|---|---|---|---|---|---|---|---|
| 17 | $CH_3$ | H | 5-tert-$C_4H_9$ | O | – | O | 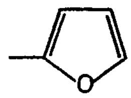 | 1,5208 |
| 18 | $CH_3$ | Cl | H | O | – | O | 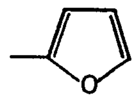 | 1,5445 |
| 19 | $CH_3$ | $CH_3$ | H | O | – | O | $CH_2OCH_3$ | |
| 20 | $CH_3$ | Cl | H | O | – | O | $CH_2OC_2H_4OCH_3$ | 1,5045 |
| 21 | $CH_3$ | $CH_3$ | 3-$CH_3$ | O | – | O | 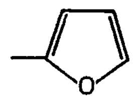 | |
| 22 | $CH_3$ | $C_2H_5$ | H | O | – | O | $CH_2OC_2H_4OCH_3$ | |
| 23 | $CH_3$ | $C_2H_5$ | H | O | – | O | 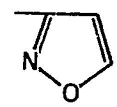 | |
| 24 | $CH_3$ | $CH_3$ | H | O | – | O | $CH_2(OC_2H_4)_2OCH_3$ | 1,4982 |
| 25 | $CH_3$ | $CH_3$ | 3-$CH_3$ | O | – | O | 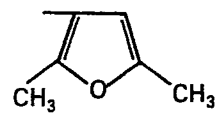 | |
| 26 | $CH_3$ | $C_2H_5$ | H | O | – | O | 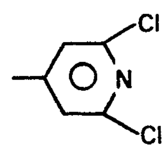 | |
| 27 | $CH_3$ | $CH_3$ | H | O | – | O | $CH_2OC_2H_4OCH_3$ | 1,4990 |
| 28 | $CH_3$ | $C_2H_5$ | H | O | – | O | $C_2H_4OC_2H_5$ | |
| 29 | $CH_3$ | $CH_3$ | H | O | – | O | 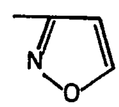 | Öl |
| 30 | $CH_3$ | $CH_3$ | H | O | – | O | 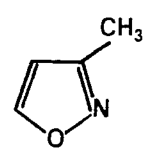 | 165–170/0,01 mbar |
| 31 | $CH_3$ | $CH_3$ | H | O | – | O | 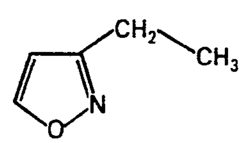 | 178–182/0,005 mbar |
| 32 | $CH_3$ | $CH_3$ | H | O | – | O | 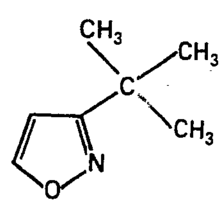 | 167–168/0,005 mbar |
| 33 | $CH_3$ | $CH_3$ | H | O | – | O | 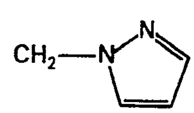 | Fp 85 |

Tabelle 2 (Fortsetzung)

| Nr. | $R^1$ | $R^2$ | $R^3$ | $X^1$ | Y | n | $R^6$ | $Kp/n_D^{20}$ |
|---|---|---|---|---|---|---|---|---|
| 34 | $CH_3$ | $CH_3$ | H | O | – | 0 | 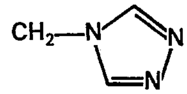 | Fp 167 |
| 35 | $CH_3$ | $CH_3$ | H | O | – | 0 | 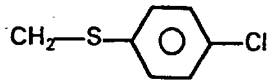 | 1,5640 |
| 36 | $CH_3$ | $C_2H_5$ | H | O | – | 0 | 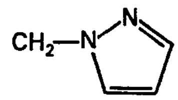 | |
| 37 | $CH_3$ | $C_2H_5$ | H | O | – | 0 | 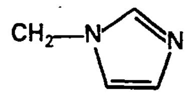 | |
| 38 | $CH_3$ | $C_2H_5$ | H | O | – | 0 | 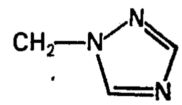 | |
| 39 | $CH_3$ | $CH_3$ | H | O | – | 0 | $CH_2\text{-}N(C_2H_5)_2$ | $n_D^{25}$= 1,5050 |
| 40 | $CH_3$ | $CH_3$ | H | O | S | 1 | $i\text{-}C_3H_7$ | 1,4984 |
| 41 | $CH_3$ | $CH_3$ | $3\text{-}CH_3$ | O | – | 0 | 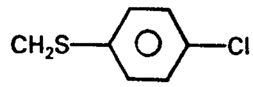 | |
| 42 | $CH_3$ | $CH_3$ | H | O | – | 0 | 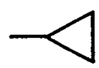 | 1,5130 |
| 43 | $CH_3$ | $CH_3$ | H | O | – | 0 | 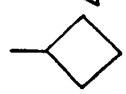 | Fp 46–52 |
| 44 | $CH_3$ | $CH_3$ | H | O | – | 0 | $CH_2\text{-}Br$ | Wachs |
| 45 | $CH_3$ | $CH_3$ | H | O | – | 0 | $CH(CH_3)\text{-}(CH_2)_2\text{-}CH_3$ | 1,4910 |
| 46 | $CH_3$ | $CH_3$ | H | O | – | 0 | 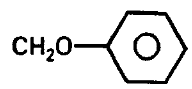 | 1,5385 |
| 47 | $CH_3$ | $CH_3$ | H | O | – | 0 | 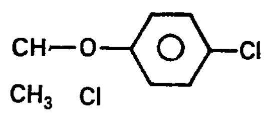 | 1,5440 |
| 48 | $CH_3$ | $CH_3$ | H | O | – | 0 | 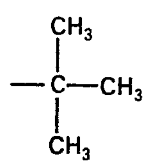 | 1,4978 |
| 49 | $CH_3$ | $CH_3$ | H | O | $N\text{-}OCH_3$ | 1 | $CH_3$ | 1,5025 |
| 50 | $CH_3$ | $C_2H_5$ | H | O | – | 0 | 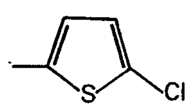 | |
| 51 | $C_2H_5$ | $CH_3$ | H | O | – | 0 | 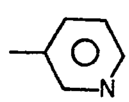 | |
| 52 | $C_2H_5$ | $CH_3$ | H | O | – | 0 | $\text{-}C(CH_3)_3$ | |
| 53 | $CH_3$ | $C_2H_5$ | H | O | – | 0 | 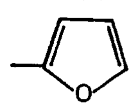 | 1,5429 |

Tabelle 2 (Fortsetzung)

| Nr. | $R^1$ | $R^2$ | $R^3$ | $X^1$ | Y | n | $R^6$ | $Kp/n_D^{20}$ |
|---|---|---|---|---|---|---|---|---|
| 54 | $C_2H_5$ | $CH_3$ | H | O | – | O | 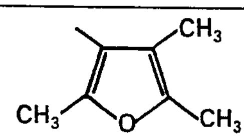 | 1,5280 |
| 55 | $CH_3$ | $C_2H_5$ | H | O | – | O | 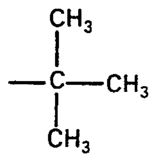 | 100–106/0,1 |
| 56 | $CH_3$ | $C_2H_5$ | H | O | O | 1 | $-C_2H_5$ | 1,4959 |
| 57 | $C_2H_5$ | $CH_3$ | H | O | O | 1 | $-CH_3$ | 1,5007 |
| 58 | $CH_3$ | $CH_3$ | 4-$CH_3$ | O | – | O | 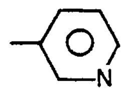 | |
| 59 | $CH_3$ | $CH_3$ | H | O | – | O | 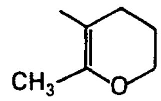 | |
| 60 | $CH_3$ | $CH_3$ | H | O | – | O | $CH_3$ | 1,5076 |
| 61 | $CH_3$ | $CH_3$ | H | O | O | 1 | 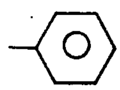 | 1,5303 |
| 62 | $CH_3$ | $CH_3$ | H | O | S | 1 | $C_2H_5$ | 1,5234 |
| 63 | $CH_3$ | $CH_3$ | H | O | O | 1 | $CH_3$ | 1,5074 |
| 64 | $CH_3$ | $CH_3$ | H | O | – | O | $CH_2$-S-$CH_3$ | |
| 65 | $CH_3$ | $CH_3$ | 4-$CH_3$ | O | – | O | $CH_2$-O$CH_3$ | 1,5028 |
| 66 | $CH_3$ | $CH_3$ | H | O | – | O | 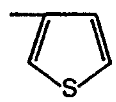 | 1,5590 |
| 67 | $CH_3$ | $CH_3$ | H | O | – | O | 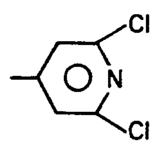 | Fp 68–70 |
| 68 | $CH_3$ | $CH_3$ | H | O | – | O | $-(CH_2)_{16}-CH_3$ | 1,4900 |
| 69 | $CH_3$ | $CH_3$ | H | O | – | O | CH=CH-$CH_3$ | Fp 72–80 |
| 70 | $CH_3$ | $CH_3$ | H | O | – | O | 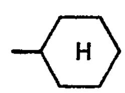 | 1,4930 |
| 71 | $CH_3$ | $CH_3$ | H | O | – | O | 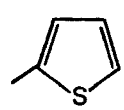 | 1,5460 |
| 72 | $CH_3$ | H | H | O | – | O | 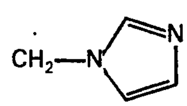 | Harz |
| 73 | $CH_3$ | Cl | H | O | – | O | 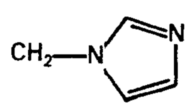 | |

Tabelle 2 (Fortsetzung)

| Nr. | $R^1$ | $R^2$ | $R^3$ | $X^1$ | Y | n | $R^6$ | $Kp/n_D^{20}$ |
|---|---|---|---|---|---|---|---|---|
| 74 | $CH_3$ | $CH_3$ | 4-$CH_3$ | O | - | 0 | 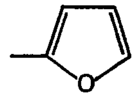 | Fp 82-86 |
| 75 | $CH_3$ | $CH_3$ | H | O | S | 1 | 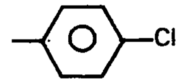 | |
| 76 | $CH_3$ | $CH_3$ | H | O | - | 0 | 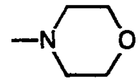 | |
| 77 | $CH_3$ | $CH_3$ | H | O | - | 0 | 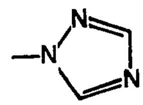 | |
| 78 | $CH_3$ | $CH_3$ | H | O | - | 0 | $CH_2$-O-$CH_3$ | 1,5071 |
| 79 | $CH_3$ | $C_2H_5$ | H | O | - | 0 | $CH_2$-O-$CH_3$ | 140-142/0,2/mbar |
| 80 | $CH_3$ | $CH_3$ | H | O | - | 0 | $CH_2$-$OC_2H_5$ | Öl |
| 81 | $CH_3$ | Cl | H | O | - | 0 | 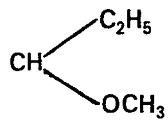 | |
| 82 | $CH_3$ | Cl | H | O | - | 0 | $CH_2$-$CH_2$-$OCH_3$ | |
| 83 | $C_2H_5$ | $CH_3$ | H | O | - | 0 | $C_2H_5$ | 1,5280 |
| 84 | $CH_3$ | $CH_3$ | H | O | - | 0 | 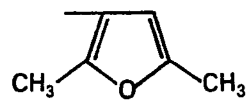 | |
| 85 | $CH_3$ | H | 5-tert-$C_4H_9$ | O | - | 0 | $CH_2$-S-$CH_3$ | |
| 86 | $CH_3$ | $CH_3$ | 4-$CH_3$ | O | - | 0 | $CH_2$-$CH_2$-$OCH_3$ | |
| 87 | $C_2H_5$ | $CH_3$ | H | O | - | 0 | $CH_2O(C_2H_4O)_2CH_3$ | |
| 88 | $CH_3$ | Cl | H | O | - | 0 | 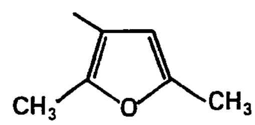 | |
| 89 | $C_2H_5$ | $C_2H_5$ | H | O | S | 1 | iso-$C_3H_7$ | |
| 90 | $CH_3$ | Cl | H | O | - | 0 | 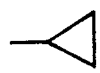 | |
| 91 | $C_2H_5$ | $C_2H_5$ | H | O | - | 0 | $CH(CH_3$-$CH_2$-$C_2H_5$ | |
| 92 | $CH_3$ | Cl | H | O | - | 0 | CH=CH-$CH_3$ | |
| 93 | $CH_3$ | $CH_3$ | 4-$CH_3$ | O | - | 0 | -$(CH_2)_{16}$-$CH_3$ | |
| 94 | $CH_3$ | Cl | H | O | S | 1 | $C_2H_5$ | |
| 95 | $CH_3$ | $CH_3$ | 4-$CH_3$ | O | O | 1 | $CH_3$ | |
| 96 | $CH_3$ | $OCH_3$ | H | O | - | 0 | $CH_2$-$OCH_3$ | |
| 97 | $CH_3$ | $CH_3$ | H | O | - | 0 | $CH(CH_3)OCH_3$ | 1,4992 |
| 98 | $CH_3$ | $CH_3$ | H | O | - | 0 | CHCl-$CH_2$-$OCH_3$ | |

Beispiel 99

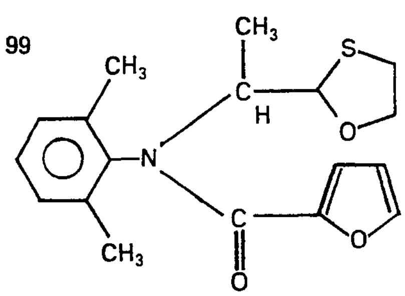

31,7 Teile 2-(N-2',6'-Dimethylphenyl-, N-furan-2''-carbonyl)-amino-propanalidimethylacetal (entsprechend Beispiel 1) werden mit 9 Teilen 2-Mercaptoethanol und einer 0,5 Teilen p-Toluolsulfonsäure für 4 Stunden bei 70°C gerührt. Dann wird das Reaktionsgemisch in 300 Teilen Methylenchlorid gelöst und zweimal mit gesättigter Natriumhydrogencarbonatlösung und zweimal mit Wasser gewaschen. Nach dem Trocknen und Abdestillieren des Lösungsmittels verbleiben 18 Teile Öl, das aus Diisopropylether kristallisiert.

Man erhält 10,2 Teile Furan-2-carbonsäure-N-(1-(1,3-oxathiolan-2-yl)-ethyl-2',6'-dimethylanilid vom Schmelzpunkt 128 - 132°C.

Entsprechend wurden folgende Verbindungen hergestellt:

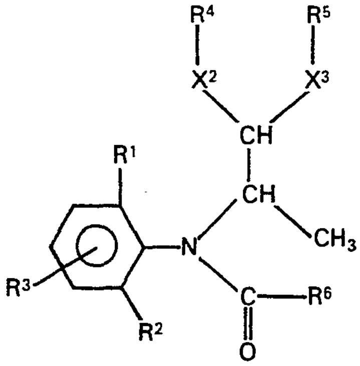

Tabelle 3

| Nr. | $R^1$ | $R^2$ | $R^3$ | $R^4$ | $R^5$ | $X^2$ | $X^3$ | $R^6$ | $Fp/n_D^{20}$ |
|---|---|---|---|---|---|---|---|---|---|
| 100 | $CH_3$ | $CH_3$ | H | $-CH_2-CH_2-$ | | O | O | 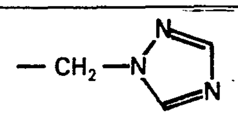 | 136 - 137 |
| 101 | $CH_3$ | $CH_3$ | H | $-CH_2-CH_2-$ | | O | O | 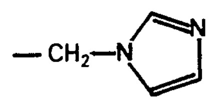 | 146 |
| 102 | $CH_3$ | $C_2H_5$ | H | $-CH_2-CH_2-$ | | O | O | 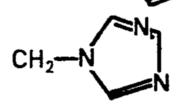 | 128 - 130 |
| 103 | $CH_2CH_3$ | $CH_3$ | H | $-CH_2-CH_2-$ | | O | O | 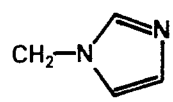 | Öl |
| 104 | $CH_3$ | $CH_3$ | H | $-CH_2-CH_2-$ | | O | O | 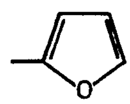 | 82 - 90 |
| 105 | $CH_3$ | $CH_3$ | H | $-CH_2-CH_2-$ | | S | O | 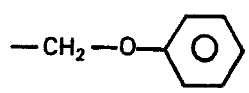 | Wachs |
| 106 | $CH_3$ | $CH_3$ | H | $-CH_2-CH_2-$ | | O | O | $CH_2-OCH_3$ | 1,5255 |
| 107 | $CH_3$ | $CH_3$ | H | $C_2H_5$ | $C_2H_5$ | O | O | $CH_2-OCH_3$ | 1,4980 |
| 108 | $CH_3$ | $CH_3$ | H | 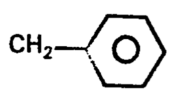 | 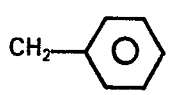 | O | O | 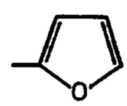 | 1,5499 |
| 109 | $CH_3$ | $CH_3$ | H | 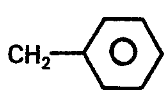 | 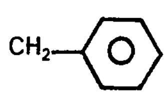 | O | O | $CH_2-OCH_3$ | 1,5370 |
| 110 | $CH_3$ | $CH_3$ | H | $-CH_2-C(CH_3)(C_6H_5)-CH_2-$ | | O | O | 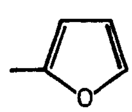 | 45 - 53 |
| 111 | $CH_3$ | $CH_3$ | H | $-CH_2-C(CH_3)(C_6H_5)-CH_2-$ | | O | O | $CH_2-OCH_3$ | 1,5470 |
| 112 | $CH_3$ | $CH_3$ | H | $-CH_2-CH_2-CH_2-$ | | O | O | 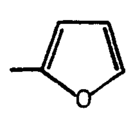 | 112 - 114 |

Tabelle 3 (Fortsetzung)

| Nr. | R[1] | R[2] | R[3] | R[4] R[5] | X[2] | X[3] | R[6] | $Fp/n_D^{20}$ |
|---|---|---|---|---|---|---|---|---|
| 113 | $CH_3$ | $CH_3$ | H | $-CH(CH_3)-CH_2-$ | O | O | 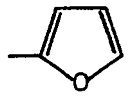 | 1,5300 |
| 114 | $CH_3$ | $CH_3$ | H | $-CH(CH_3)-CH(CH_3)-$ | O | O | 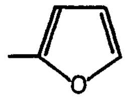 | 1,5320 |
| 115 | $CH_3$ | $CH_3$ | H | $-CH(CH_3)-CH_2-CH_2-$ | O | O | 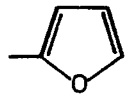 | 1,5255 |
| 116 | $CH_3$ | $CH_3$ | $4-CH_3$ | $-CH_2-CH_2-CH_2-$ | O | O | 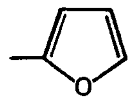 | Öl |
| 117 | $CH_3$ | $CH_3$ | H | $-CH(CH_3)-CH_2-$ | O | O | $CH_2-OCH_3$ | 1,5182 |
| 118 | $CH_3$ | $CH_3$ | H | $-CH_2-CH_2-CH_2-$ | O | O | $CH_2-OCH_3$ | 85 - 90 |
| 119 | $CH_3$ | $CH_3$ | H | $-CH(CH_3)-CH_2-CH_2$ | O | O | $CH_2-OCH_3$ | 1,4980 |
| 120 | $CH_3$ | $CH_3$ | $4-CH_3$ | $-CH_2-CH_2-$ | O | O | $CH_2-OCH_3$ | Öl |
| 121 | $CH_3$ | $CH_3$ | H | $-CH(CH_3)-CH(CH_3)-$ | O | O | $CH_2-OCH_3$ | 1,5075 |
| 122 | $CH_3$ | Cl | H | $-CH_2-CH_2-$ | O | O | $CH_2-OCH_3$ | 1,5298 |
| 123 | $CH_3$ | $CH_3$ | H | $-CH_2-CH_2-$ | S | O | $CH_2-OCH_3$ | |
| 124 | $CH_3$ | $CH_3$ | H | $-CH_2-C(CH_3)_2-CH_2-$ | O | O | 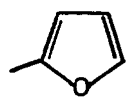 | 103 - 105 |
| 125 | $CH_3$ | $CH_3$ | H | $-CH_2-C(CH_3)_2-CH_2-$ | O | O | $CH_2-OCH_3$ | 1,5121 |
| 126 | $CH_3$ | $CH_3$ | H | $-CH_2-CH_2-$ | O | O | 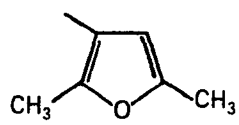 | 96 - 102 |
| 127 | $CH_3$ | $CH_3$ | H | $-CH_2-C(CH_3)(C_2H_5)-CH_2-$ | O | O | $CH_2-S-CH_3$ | |
| 128 | $CH_3$ | $CH_3$ | H | $-CH_2-CH_2-$ | O | O | $CH_2-S-CH_3$ | |
| 129 | $CH_3$ | $CH_3$ | H | $-CH(C_2H_5)-CH_2-$ | O | O | $CH_2-O-CH_3$ | 1,5127 |
| 130 | $CH_3$ | $CH_3$ | H | $-CH_2-C(C_2H_5)(n-C_4H_9)-CH_2-$ | O | O | $CH_2-O-CH_3$ | 105 - 109 |
| 131 | $CH_3$ | H | H | $-CH_2-CH_2-$ | O | O | $CH_2-OCH_3$ | Öl |
| 132 | $CH_3$ | $CH_3$ | H | $-CH_2-CH(C_6H_5)-$ | O | O | $CH_2-OCH_3$ | 1,543 |
| 133 | $CH_3$ | $CH_3$ | H | $-CH_2-C(CH_3)(nC_3H_7)-CH_2-$ | O | O | $CH_2-OCH_3$ | 95 -97 |
| 134 | $CH_3$ | Cl | H | $-CH_2-C(CH_3)(C_6H_5)CH_2$ | O | O | $CH_2-OCH_3$ | 1.5448 |
| 135 | $CH_3$ | $CH_3$ | H | $-CH_2-CH_2$ | O | O | $CH_2-OC_2H_5$ | 1.5145 |
| 136 | $CH_3$ | Cl | H | $-CH_2-CH_2-$ | O | O | $CH_2OC_2H_4OCH_3$ | |
| 137 | $CH_3$ | $CH_3$ | H | $-CH_2-CH_2-$ | O | O | $C_2H_4-O-CH_3$ | 1.5130 |
| 138 | $CH_3$ | Cl | H | $-CH_2-CH_2-$ | O | O | $CH_2-(OC_2H_4)_2OCH_3$ | 1.5177 |
| 139 | $CH_3$ | $CH_3$ | H | $-CH_2-CH_2-$ | O | O | $CH_2OC_2H_4OCH_3$ | 1.5138 |
| 140 | $CH_3$ | $CH_3$ | H | $-CH_2-CH_2-$ | O | O | $CH(CH_3)OCH_3$ | 1.5130 |
| 141 | $CH_3$ | $CH_3$ | H | $-CH_2-CH_2-$ | O | O | $CH_2-(OC_2H_4)_2-OCH_3$ | 1.5135 |
| 142 | $CH_3$ | $CH_3$ | H | $-CH_2-CH_2-$ | O | O | 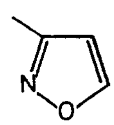 | 1.5386 |
| 143 | $CH_3$ | $CH_3$ | H | $-CH_2-CH_2-$ | O | O | 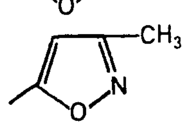 | 1.5362 |

Tabelle 3 (Fortsetzung)

| Nr. | $R^1$ | $R^2$ | $R^3$ | $R^4$ | $R^5$ | $X^2$ | $X^3$ | $R^6$ | $Fp/n_D^{20}$ |
|---|---|---|---|---|---|---|---|---|---|
| 144 | $CH_3$ | $CH_3$ | 4-$CH_3$ | $-CH_2-CH_2-$ | | O | O | 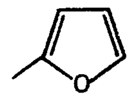 | Öl |
| 145 | $CH_3$ | $CH_3$ | H | $-CH_2-CH_2-$ | | O | O | 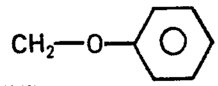 | 64–70°C |

Die erfindungsgemässen Wirkstoffe weisen eine starke fungitoxische Wirkung auf. Sie schädigen Kulturpflanzen in den zur Bekämpfung von Pilzen und Bakterien notwendigen Konzentrationen nicht. Aus diesen Gründen sind sie für den Gebrauch als Pflanzenschutzmittel zur Bekämpfung von Pilzen geeignet.

Die neuen Wirkstoffe zeigen eine starke fungitoxische Wirksamkeit gegen phytopathogene Pilze, insbesondere aus der Klasse der Phycomyceten. Die beanspruchten Verbindungen sind daher beispielsweise geeignet zur Bekämpfung von Phytophthora infestans an Tomaten und Kartoffeln, Phytophthora parasitica an Erdbeeren, Phytophthora cactorum an Äpfeln, Pseudoperonospora cubensis an Gurken, Pseudoperonospora humuli an Hopfen, Peronospora destructor an Zwiebeln, Peronospora sparsa an Rosen, Peronospora tabacina an Tabak, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben. Die fungiziden Mittel enthalten 0,1 bis 95% (Gewichtsprozent) Wirkstoff, vorzugsweise 0,5 bis 90%. Die Aufwandmengen liegen je nach Art des gewünschten Effektes zwischen 0,1 und 5 kg Wirkstoff je ha. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen. Darüber hinaus sind viele der neuen Verbindungen systemisch wirksam, so dass über die Wurzelbehandlung auch ein Schutz oberirdischer Pflanzenteile möglich ist. Ferner lassen sich mit den neuen Verbindungen auch Pilze, die Keimlings- und Auflaufkrankheiten hervorrufen, beispielsweise Pythium- und Aphanomyces-Arten an Leguminosen und Baumwolle, bekämpfen. Die Aufwandmengen betragen je 100 kg Saatgut 10 bis 200 g Wirkstoff; die Anwendung erfolgt in Form von Saatgutbeizmitteln.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die erfindungsgemässen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollten in jedem Fall eine feine und gleichmässige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen – Fettalkohol – Ether, Alkylsulfonate und Arylsulfonate) und Dispergiermittel, wie Lignin, Sulfitablaugen und Methylcellulose.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Giessen.

Beispiele für solche Zubereitungen sind:

I. Man vermischt 90 Gewichtsteile der Verbindung des Beispiels 1 mit 10 Gewichtsteilen N-Methyl- -pyroliden und erhält eine Lösung, die zur Anwendung in Form kleinster Tropfen geeignet ist.

II. 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgiessen und feines Verteilen der Lösung in 100 000 Gewichtsteilen Wasser erhält man eine wässrige Dispersion, die

0,02 Gew.% des Wirkstoffs enthält.

III. 20 Gewichtsteile der Verbindung des Beispiels 3 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingiessen und feines Verteilen der Lösung in 100000 Gewichtsteilen Wasser enthält man eine wässrige Dispersion, die 0,02 Gew.% des Wirkstoffs enthält.

IV. 20 Gewichtsteile der Verbindung des Beispiels 1 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingiessen und feines Verteilen der Lösung in 100000 Gewichtsteilen Wasser erhält man eine wässrige Dispersion, die 0,02 Gewichtsprozent des Wirkstoffs enthält.

V. 20 Gewichtsteile der Verbindung des Beispiels 2 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselsäureregel gut vermischt und in einer Hammermühle vermahlen. Durch feines Verteilen der Mischung in 20000 Gewichtsteilen Wasser erhält man eine Spritzbrühe, die 0,1 Gew.% des Wirkstoffs enthält.

VI. 3 Gewichtsteile der Verbindung des Beispiels 3 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung des Beispiels 1 werden mit einer Mischung aus 92 Gewichtsteilen pulverförmigem Kieselsäureregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung des Beispiels 2 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wässrige Dispersion. Durch Verdünnen mit 100000 Gewichtsteilen Wasser erhält man eine wässrige Dispersion, die 0,04 Gew.% Wirkstoff enthält.

IX. 20 Teile der Verbindung des Beispiels 3 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemässen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrösserung des fungiziden Wirkungsspektrums.

Die folgende Liste von Fungiziden, mit denen die erfindungsgemässen Verbindungen kombiniert werden können, soll die Kombinationsmöglichkeiten erläutern, nicht aber einschränken.

Fungizide, die mit den erfindungsgemässen Verbindungen kombiniert werden können, sind beispielsweise:

Dithiocarbamate und deren Derivate, wie
Ferridimethyldithiocarbamat,
Zinkdimethyldithiocarbamat,
Manganethylenbisdithiocarbamat,
Mangan-Zink-ethylendiamin-bis-dithiocarbamat,
Zinkethylenbisdithiocarbamat,
Tetramethylthiuramdisulfide,
Ammoniak-Komplex von Zink-(N,N-ethylen-bis-dithiocarbamat) und
N,N'-Polyäthylen-bis-(thiocarbamoyl)-disulfid;
Zink-(N,N'-propylen-bis-dithiocarbamat), Ammoniak-Komplex von Zink-(N,N'-propylen-bis-dithiocarbamat) und N,N'-Polypropylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Strukturen, wie
N-Trichlormethylthio-tetrahydrophthalimid,
N-Trichlormethylthio-phthalimid,
2-Heptadecyl-2-imidazolin-acetat,
2,4-Dichlor-6-(o-chloranilino)-s-triazin,
0,0-Diäthyl-phthalimidophosphonothioat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-l-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,

Piperazin-1,4-diyl-bis(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-Chlorphenyl)-3-pyridinmethanol,
1,2-Bis-(3-ethoxycarbonyl-2-thioureido)-benzol,
1,2-Bis-(3-methoxycarbonyl-2-thioureido)-benzol

und verschiedene Fungizide, wie
Dodecylguanidinacetat,
3-(3-(3,5-Dimethyl-2-oxycyclohexyl)-2-hydroxyäthyl)-glutarimid,
Hexachlorbenzol,
N-Dichlorfluormethylthio-N', N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Methyl-benzoesäure-anilid,
2-Jod-benzoesäure-anilid,
1-(3,4-Dichloranilino)-1-formylamino-2,2,2-trichloräthan,
2,6-Dimethyl-N-tridecyl-morpholin bzw. dessen Salze,
2,6-Dimethyl-N-cyclododecyl-morpholin bzw. dessen Salze.
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
5-Nitro-isophthalsäure-di-isopropylester,
1-(1',2',4'-Triazolyl-1')-[1-(4'-chlorphenoxy)]-3,3-dimethylbutan-2-on,
1-(1',2',4'-Triazolyl-1')-[1-(4'-chlorphenoxy)]-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazolyl-harnstoff,
N-Cyclohexyl-N-methoxy-2,5-dimethyl-furan-3-carbonsäureamid,
2,4,5-Trimethyl-furan-3-carbonsäureanilid,
5-Methyl-5-vinyl-3-(3,5-dichlorphenyl)-2,4-dioxo-1,3-oxazolidin,
5-Methoxymethyl-5-methyl-3-(3,5-dichlorphenyl)-2,4-dioxo-1,3-oxazolidin,
N-[3-(p-tert.-Butylphenyl)-2-methyl-propyl]-cis-2,6-dimethylmorpholin.

Für die folgenden Versuche wurden als bekannte Vergleichswirkstoffe die folgenden Verbindungen verwendet.

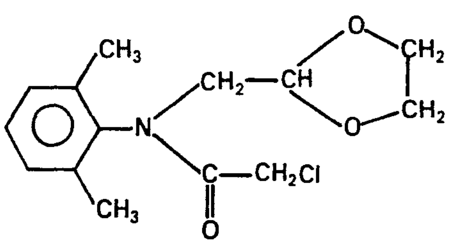

(Verbindung A)
bekannt aus
DE-OS 24 05 510

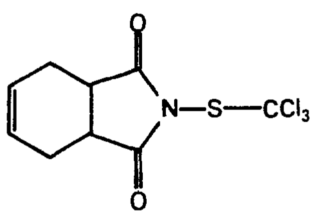

(Verbindung B)
bekannt aus
Chem. Week 1972,
June 21, Seite 63.

Versuch 1

Fungizide Wirksamkeit gegen Phytophthora infestans an Tomaten

Blätter von Tomatenpflanzen der Sorte «Professor Rudloff» werden mit wässrigen Suspensionen, die 80% (Gewichtsprozent) des zu prüfenden Wirkstoffes und 20% Natriumligninsulfonat in der Trockensubstanz enthalten, besprüht. Es werden 0,025 und 0,12%ige (Gew.%) Spritzbrühen (berechnet auf die Trockensubstanz) verwendet. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, dass die fungizide Wirksamkeit der Substanzen beurteilt werden kann:

0 = kein Pilzbefall, abgestuft bis 5 = Totalbefall (Kontrolle)

| Wirkstoff | Befall der Blätter nach Spritzung mit ..%iger Wirkstoffbrühe | |
|---|---|---|
| | 0,025 | 0,012 |
| 2 | 0 | 0 |
| 6 | 0 | 2 |
| 9 | 0 | 2 |
| 33 | 0 | 1 |
| 34 | 0 | 1 |
| 104 | 0 | 0 |

| Wirkstoff | Befall der Blätter nach Spritzung mit ..%iger Wirkstoffbrühe 0,025 | 0,012 |
|---|---|---|
| 111 | 0 | 2 |
| 20 | 1 | 2-3 |
| 24 | 1 | 2 |
| 27 | 1 | 2 |
| 80 | 0 | 1 |
| 101 | 1 | 2 |
| 102 | 0 | 2 |
| 117 | 0 | 2 |
| 118 | 1 | 2 |
| 119 | 0 | 2-3 |
| Kontrolle (unbehandelt) | | 5 |

Versuch 2
Fungizide Wirksamkeit gegen Auflaufkrankheiten an Erbsen

100 g-Proben Erbsensamen der Sorte «Senator» werden in Glasflaschen etwa 5 Minuten lang mit 300 mg (= 0,3 Gew.%) Beizmittelaufbereitungen, die 40% (Gew.%) Wirkstoff in der Trockensubstanz enthalten, sorgfältig geschüttelt. Danach werden jeweils 100 Samen in Saatkisten 3 cm tief und mit einem Abstand von 3 bis 5 cm in eine Komposterde eingesät, die eine starke natürliche Verseuchung mit den Pilzen Pythium spec., Aphanomyces spec. und Fusarium oxysporum aufweist. Die Kästen werden im Gewächshaus bei Temperaturen von 17 bis 20°C aufgestellt. Nach einer Versuchsdauer von 21 Tagen wird die Anzahl gesunder Erbsenpflanzen ermittelt.

| Wirkstoff | .. % gesunde Pflanzen nach 21 Tagen in Komposterde |
|---|---|
| 20 | 96 |
| 42 | 96 |
| 78 | 94 |
| A (bekannt) | 15 |
| B (bekannt) | 63 |
| Kontrolle (unbehandelt) | 8 |
| Kontrolle (sterilisierte Komposterde) | 98 |

**Patentansprüche**
für die Vertragsstaaten BE, CH, LI, DE, FR, GB, IT, LU, NL, SE

1. 2-(N-Aryl-, N-acyl)-aminopropanalacetale der allgemeinen Formel I

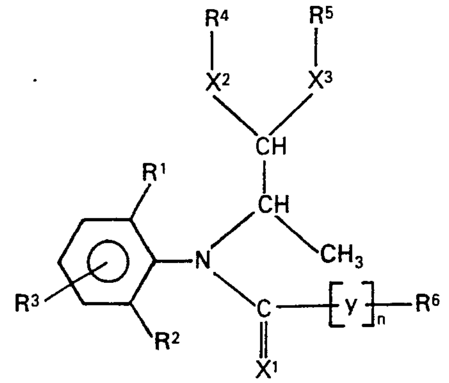

worin
$X^1$, $X^2$ und $X^3$ unabhängig voneinander Sauerstoff oder Schwefel,
Y Sauerstoff, Schwefel oder $NR^7$,
n 0 oder 1,
$R^1$ $C_1$-$C_4$-Alkyl,
$R^2$ Wasserstoff, Halogen, $C_1$–$C_4$-Alkyl oder $C_1$–$C_4$-Alkoxy,
$R^3$ Wasserstoff, Halogen oder $C_1$–$C_4$-Alkyl,
$R^4$ und $R^5$ unabhängig voneinander gegebenenfalls substituierte Alkyl-oder Arylalkylreste bedeuten oder zusammen als Alkylgruppe Teil eines gegebenenfalls durch Alkyl oder Aryl substituierten 5- oder 6-gliedrigen heterocyclischen Ringes sind,
$R^6$ einen gegebenenfalls substituierten Alkyl-, Alkenyl-, Cycloalkyl-, Cycloalkenyl- oder Heteroarylrest mit Ausnahme des Chlormethylrestes und des Acetylmethylrestes bedeutet und für den Fall, dass n = 1 ist, $R^6$ zusätzlich zu den oben genannten Bedeutungen einen gegebenenfalls substituierten Alkinyl-, Aryl-, Arylalkyl- oder Heteroarylalkylrest bedeutet und
$R^7$ Wasserstoff, eine Alkyl- oder Alkoxygruppe bedeutet.

2. Fungizid, enthaltend ein 2-(N-Aryl-, N-acyl)-aminopropanalacetal gemäss Anspruch 1.

3. Fungizid, enthaltend einen festen oder flüssigen Trägerstoff und ein 2-(N-Aryl-, N-acyl)-aminopropanalacetal gemäss Anspruch 1.

4. Verfahren zur Herstellung eines Fungizids, dadurch gekennzeichnet, dass man einen festen oder flüssigen Trägerstoff vermischt mit einem 2-(N-Aryl-, N-acyl)-amino-propanalacetal gemäss Anspruch 1.

5. Verfahren zur Bekämpfung von Pilzen, dadurch gekennzeichnet, dass man die Pilze oder die vor Pilzbefall zu schützenden Gegenstände behandelt mit einem 2-(N-Aryl-,N-acyl)-amino-propanalacetal gemäss Anspruch 1.

6. Verfahren zur Herstellung eines 2-(N-Aryl-, N-acyl)-aminopropanalacetals gemäss Anspruch 1, dadurch gekennzeichnet, dass man ein Anilin der Formel

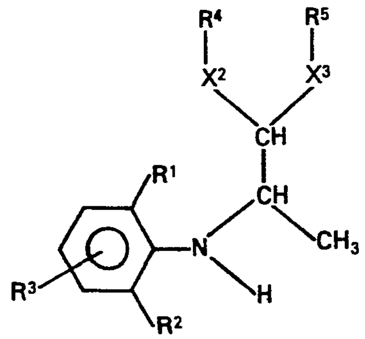

worin $R^1$, $R^2$, $R^3$, $R^4$, $R^5$, $X^2$ und $X^3$ die oben angegebenen Bedeutungen haben,

a) mit einem Säurehalogenid der Formel

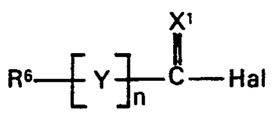

oder

b) mit einem Säureanhydrid der Formel

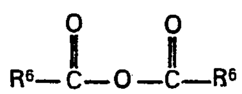

oder

c) mit einem Isocyanat der Formel

$R^6{-}N{=}C{=}X^1$

worin $X^1$, Y, n und $R^6$ die oben angegebenen Bedeutungen haben, gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 0 und 120 °C umsetzt.

7. Verfahren zur Herstellung eines 2-(N-Aryl-, N-acyl)-aminopropanalacetals gemäss Anspruch 1, dadurch gekennzeichnet, dass man ein Anilin der Formel

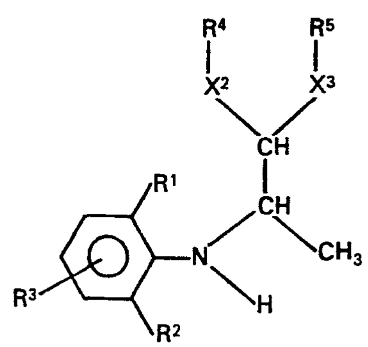

worin $R^1$, $R^2$, $R^3$, $R^4$, $R^5$, $X^2$ und $X^3$ die oben angegebenen Bedeutungen haben, zunächst

a) mit einem Phosgen der Formel

$X^1 = CCl_2$

worin $X^1$ die oben angegebene Bedeutung hat, zu einer Verbindung der Formel

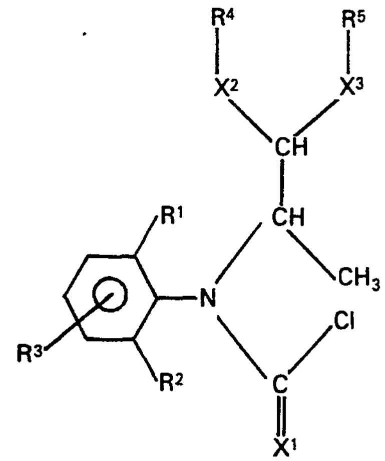

umsetzt

b) mit einer Verbindung der Formel

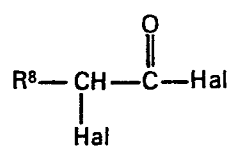

worin $R^8$ für Wasserstoff steht oder die für $R^6$ angegebenen Bedeutungen hat und Hal ein Halogenatom bedeutet, zu einer Verbindung der Formel umsetzt

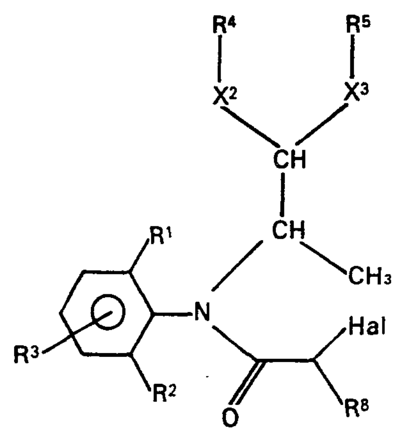

und dann die oben genannten Verbindungen mit nucleophilen Verbindungen der Formel

$R^6{-}YH$

worin Y und $R^6$ die oben angegebenen Bedeutungen haben, gegebenenfalls in Gegenwart eines Lösungs- oder Verdünnungsmittels, gegebenenfalls in Gegenwart einer anorganischen oder organischen Base, gegebenenfalls unter Zusatz eines Reaktionsbeschleunigers bei Temperaturen zwischen -20 und +100 °C umsetzt.

8. Verfahren zur Herstellung eines 2-(N-Aryl-, N-acyl)-aminopropanalacetals gemäss Anspruch 1, dadurch gekennzeichnet, dass man Verbindungen der Formel

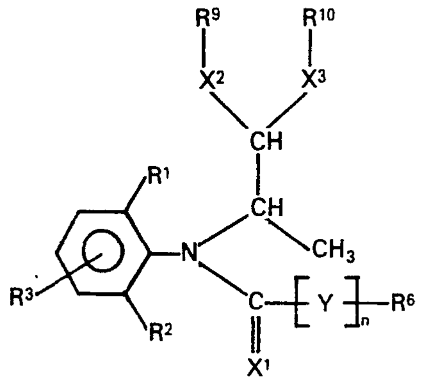

worin $R^1$, $R^2$, $R^3$, $R^6$, $X^1$, $X^2$, $X^3$, Y und n die oben genannten Bedeutungen haben und $R^9$ und $R^{10}$ unabhängig voneinander gegebenenfalls substituierte Alkyl- oder Arylalkylreste bedeuten, mit geeigneten 1,2- oder 1,3-Diolen, -Dithiolen oder -Mercaptoalkoholen bei Temperaturen zwischen 0 und 120 °C zu cyclischen Acetalen gegebenenfalls in Gegenwart eines Lösungs- oder Verdünnungsmittels und gegebenenfalls unter Zusatz eines Reaktionsbeschleunigers bei Temperaturen zwischen 0 und 120 °C umacetalisiert.

9. 2-(N-Aryl-, N-acyl)-aminopropanalacetal gemäss Anspruch 1 ausgewählt aus der Gruppe, bestehend aus 2-(N-2′,6′-Dimethylphenyl-, N-methoxyacetyl)-aminopropanaldimethylacetal, 2-(N-2′,6′-Dimethylphenyl-, N-furan-2″-carbonyl)-aminopropanaldimethylacetal, 2-Methoxyessigsäure-N-(1-(1,3-dioxolan-2-yl)-ehtyl)-2′,6′-dimethylanilid, Furan-2-carbonsäure-N-(1-(1,3-dioxolan-2-yl)-ethyl)-2′,6′-dimethylanilid und 2-Methoxyessigsäure-N-(1-(5-methyl-5-phenyl-1,3-dioxan-2-yl)-ethyl)-2′,6′-dimethylanilid.

10. Fungizid gemäss Anspruch 2, enthaltend ein 2-(N-Aryl-, N-acyl)-aminopropanalacetal ausgewählt aus der Gruppe bestehend aus 2-(N2′,6′-Dimethylphenyl-, N-methoxyacetal)-aminopropanaldimethylacetal, 2-(N-2′,6′-Dimethylphenyl-, N-furan-2″-carbonyl)-aminopropanaldimethylacetal, 2-Methoxyessigsäure-N-(1-(1,3-dioxolan-2-yl)-ethyl)-2′,6′-dimethylanilid, Furan-2-carbonsäure-N-(1-(1,3-dioxolan-2-yl)- -ehtyl)-2′,6′-dimethylanilid und 2-Methoxyessigsäure-N-(1-(5-methyl-5-phenyl-1,3-dioxan-2-yl)-ethyl)-2′,6′-dimethylanilid.

**Patentansprüche**
für den Vertragsstaat AT

1. Fungizid, enthaltend ein 2-(N-Aryl-, N-acyl)-aminopropanalacetal der Formel

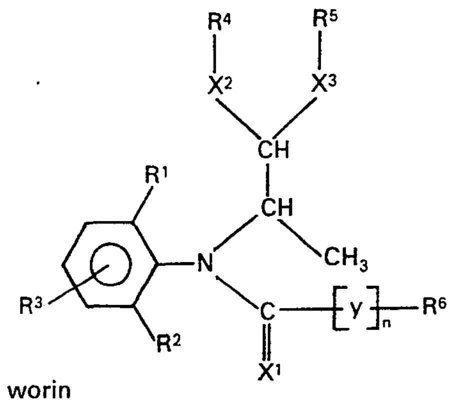

worin
$X^1$, $X^2$ und $X^3$ unabhängig voneinander Sauerstoff oder Schwefel,
Y Sauerstoff, Schwefel oder $NR^7$,
n 0 oder 1,
$R^1$ $C_1$-$C_4$-Alkyl,
$R^2$ Wasserstoff, Halogen, $C_1$-$C_4$-Alkyl oder $C_1$-$C_4$-Alkoxy,
$R^3$ Wasserstoff, Halogen oder $C_1$-$C_4$-Alkyl,
$R^4$ und $R^5$ unabhängig voneinander gegebenenfalls substituierte Alkyl-oder Arylalkylreste bedeuten oder zusammen als Alkylgruppe Teil eines gegebenenfalls durch Alkyl oder Aryl substituierten 5- oder 6-gliedrigen heterocyclischen Ringes sind,
$R^6$ einen gegebenenfalls substituierten Alkyl-, Alkenyl-, Cycloalkyl-, Cycloalkenyl- oder Heteroarylrest mit Ausnahme des Chlormethylrestes und des Acetylmethylrestes bedeutet und für den Fall, dass n = 1 ist, $R^6$ zusätzlich zu den oben genannten Bedeutungen einen gegebenenfalls substituierten Alkinyl-, Aryl-, Arylalkyl- oder Heteroarylalkylrest bedeutet und $R^7$ Wasserstoff, eine Alkyl- oder Alkoxygruppe bedeutet.

2. Fungizid, enthaltend einen festen oder flüssigen Trägerstoff und ein 2-(N-Aryl-, N-acyl)-aminopropanalacetal wie in Anspruch 1 definiert.

3. Verfahren zur Herstellung eines Fungizids, dadurch gekennzeichnet, dass man einen festen oder flüssigen Trägerstoff vermischt mit einem 2-(N-Aryl-, N-acyl)-aminopropanalacetal wie in Anspruch 1 definiert.

4. Verfahren zur Bekämpfung von Pilzen, dadurch gekennzeichnet, dass man die Pilze oder die vor Pilzbefall zu schützenden Gegenstände behandelt mit einem 2-(N-Aryl-, N-acyl)-aminopropanalacetal wie in Anspruch 1 definiert.

5. Verfahren zur Herstellung eines 2-(N-Aryl-, N-acyl)-aminopropanalacetals wie in Anspruch 1 definiert, dadurch gekennzeichnet, dass man ein Anilin der Formel

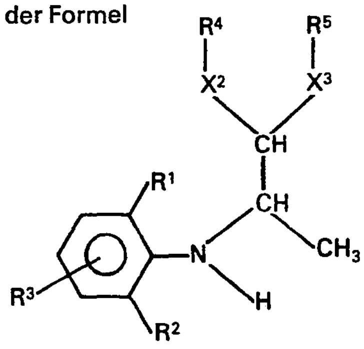

worin $R^1$, $R^2$, $R^3$, $R^4$, $R^5$, $X^2$ und $X^3$ die oben angegebenen Bedeutungen haben,

a) mit einem Säurehalogenid der Formel

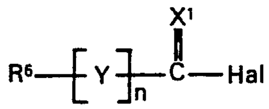

oder

b) mit einem Säureanhydrid der Formel

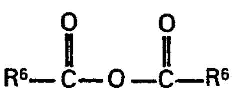

oder

c) mit einem Isocyanat der Formel

$R^6$-N=C=$X^1$

worin $X^1$, Y, n und $R^6$ die oben angegebenen Bedeutungen haben, gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 0 und 120 °C umsetzt.

6. Verfahren zur Herstellung eines 2-(N-Aryl-, N-acyl)-aminopropanalacetals wie in Anspruch 1 definiert, dadurch gekennzeichnet, dass man ein Anilin der Formel

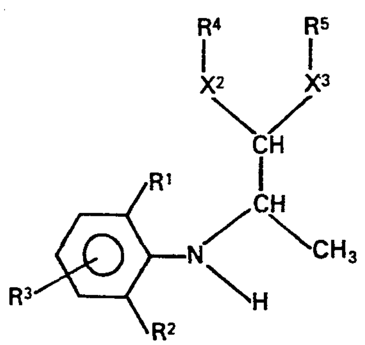

worin $R^1$, $R^2$, $R^3$, $R^4$, $R^5$, $X^2$ und $X^3$ die oben angegebenen Bedeutungen haben, zunächst

a) mit einem Phosgen der Formel VI

$X^1 = CCl_2$

worin $X^1$ die oben angegebene Bedeutung hat, zu einer Verbindung der Formel

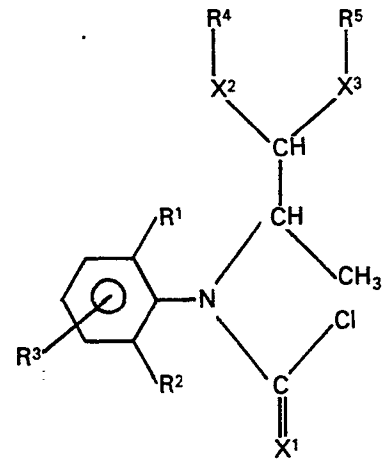

umsetzt

b) mit einer Verbindung der Formel

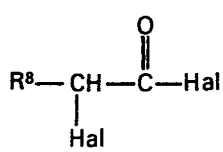

worin $R^8$ für Wasserstoff steht oder die für $R^6$ angegebenen Bedeutungen hat und Hal ein Halogenatom bedeutet, zu einer Verbindung der Formel umsetzt

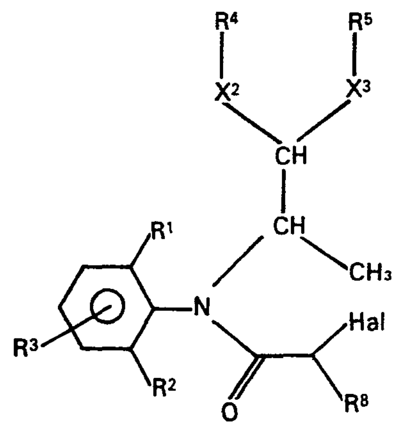

und dann die oben genannten Verbindungen der Formeln mit nucleophilen Verbindungen der Formel

$R^6$-YH

worin Y und $R^6$ die oben angegebenen Bedeutungen haben, gegebenenfalls in Gegenwart eines Lösungs- oder Verdünnungsmittels, gegebenenfalls in Gegenwart einer anorganischen oder organischen Base, gegebenenfalls unter Zusatz eines Reaktionsbeschleunigers bei Temperaturen zwischen −20 und +100 °C umsetzt.

7. Verfahren zur Herstellung eines 2-(N-Aryl-, N-acyl)-aminopropanalacetals wie in Anspruch 1 definiert, dadurch gekennzeichnet, dass man Verbindungen der Formel

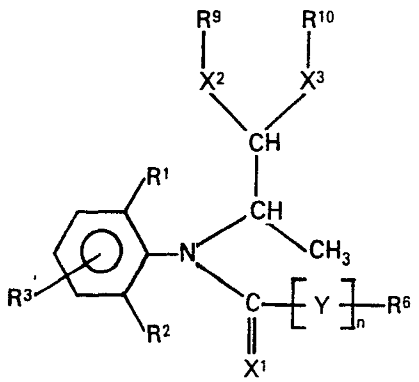

worin $R^1$, $R^2$, $R^3$, $R^6$, $X^1$, $X^2$, $X^3$, Y und n die oben genannten Bedeutungen haben und $R^9$ und $R^{10}$ unabhängig voneinander gegebenenfalls substituierte Alkyl- oder Arylalkylreste bedeuten, mit geeigneten 1,2- oder 1,3-Diolen, -Dithiolen oder -Mercaptoalkoholen bei Temperaturen zwischen 0 und 120 °C zu cyclischen Acetalen gegebenenfalls in Gegenwart eines Lösungs- oder Verdünnungsmittels und gegebenenfalls unter Zusatz eines Reaktionsbeschleunigers bei Temperaturen zwischen 0 und 120 °C umacetalisiert.

8. Fungizid, gemäss Anspruch 1, enthaltend ein 2-(N-Aryl-, N-acyl)-aminopropanalacetal ausgewählt aus der Gruppe bestehend aus 2-(N-2′,6′-Dimethylphenyl-, N-methoxyacetyl)-aminopropanaldimethylacetal, 2-(N-2′,6′-Dimenthylphenyl-, N-furan-2″-carbonyl)-aminopropanaldimethylacetal, 2-Methoxyessigsäure-N-(1-(1,3-dioxolan-2-yl)-ethyl)-2′,6′-dimethylanilid, Furan-2-carbonsäure-N-(1-(1,3-dioxolan-2-yl)-ethyl)-2′,6′-dimethylanilid und 2-Methoxyessigsäure-N-(1-(5-methyl-5-phenyl-1,3-dioxan-2-yl)-ethyl-2′,6′-dimethylanilid.

**Claims**

for the Contracting States: BE, CH, LI, DE, FR, GB, IT, LU, NL, SE

1. A 2-(N-aryl-n-acyl)-aminopropanal-acetal of the general formula I

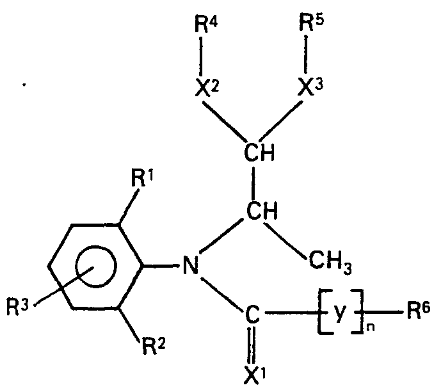

where

$X^1$, $X^2$ and $X^3$ independently of one another are oxygen or sulfur, Y is oxygen, sulfur or $NR^7$,

n is 0 or 1,

$R^1$ is $C_1$-$C_4$-alkyl,

$R^2$ is hydrogen, halogen, $C_1$-$CV_4$-alkyl or $C_1$-$C_4$-alkoxy, $R^3$ ishydrogen, halogen or $C_1$-$C_4$-alkyl,

$R^4$ and $R^5$ independently of one another are unsubstituted or substituted alkyl or arylalkyl or together are alkylene and form part of a 5-membered or 6-membered heterocyclic ring which may or may not be substituted by alkyl or aryl,

$R^6$ is unsubstituted or substituted alkyl, alkenyl, cycloalkyl, cycloalkenyl or heteroaryl, whith the exception of chloromethyl and acetylmethyl, and if

n is 1, $R^6$ may also be unsubstituted or substituted alkynyl, aryl, arylalkyl or heteroarylalkyl, and

$R^7$ is hydrogen, alkyl or alkoxy.

2. A fungicide containing a 2-(N-aryl-N-acyl)-aminopropanal-acetal as claimed in claim 1.

3. A fungicide containing a solid or liquid carrier and a 2-(N-aryl-N-acyl)aminopropanal-acetal as claimed in claim 1.

4. A process for manufacturing a fungicide, characterized in that a solid or liquid carrier is mixed with a 2-(N-aryl-N-acyl)-aminopropanal-acetal as claimed in claim 1.

5. A process for combating fungi, characterized in that the fungi or the objects to be protected against fungus attack are treated with a 2-(N-aryl-N-acyl)-aminopropanal-acetal as claimed in claim 1.

6. A process for the manufacture of a 2-(N-aryl-N-acyl)-aminopropanal-acetal as claimed in claim 1. characterized in that an aniline of the formula

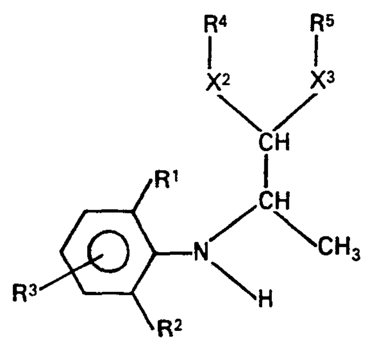

where $R^1$, $R^2$, $R^3$, $R^4$, $R^5$, $X^2$ and $X^3$ have the above meanings, is reacted

a) with an acid halide of the formula

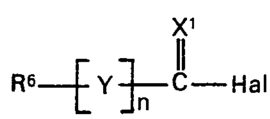

or

b) with an acid anhydride of the formula

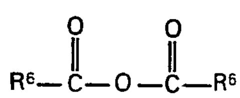

or

c) with an isocyanate of the formula

$R^6-N=C=X^1$,

where $X^1$, Y, n and $R^6$ have the above meanings, in the presence or absence of a solvent or diluent, with or without addition of an inorganic or or organic base, and with or without addition of a reaction accelerator, at from 0 to 120°C.

7. A process for the manufacture of a 2-(N-aryl-N-acyl)aminopropanal-acetal as claimed in claim 1, characterized in that an aniline of the formula

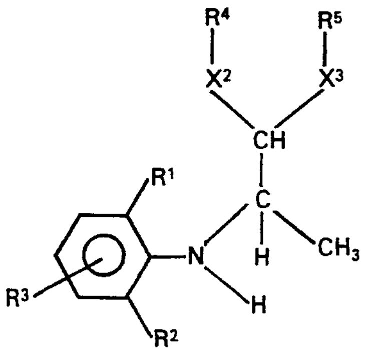

where $R^1$, $R^2$, $R^3$, $R^4$, $R^5$, $X^2$ and $X^3$ have the above meanings, is first reacted

a) with a phosgene of the formua

$X^1 = CCl_2$,

where $X^1$ has the above meaning, to give a compound of the formula

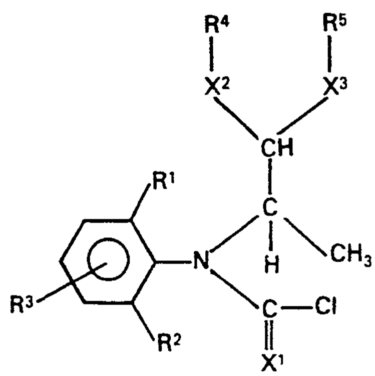

or

b) with a compound of the formula

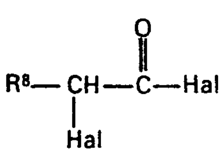

where $R^8$ is hydrogen or has the meanings given for $R^6$ and Hal is halogen, to give a compound of the formula

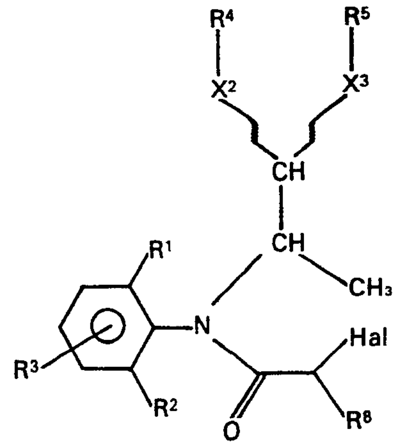

after which the above compounds are reacted with a nucleophilic compound of the formula

$R^6$-YH,

where Y and $R^6$ have the above meanings, in the presence or absence of a solvent or diluent, in the presence or absence of an inorganic or organic base, and with or without addition of a reaction accelerator, at from -20 to +100°C.

8. A process fot the manufacture of a 2-(N-aryl-N-acyl)aminopropanal-acetal as claimed in claim 1, characterized in that a compound of the formula

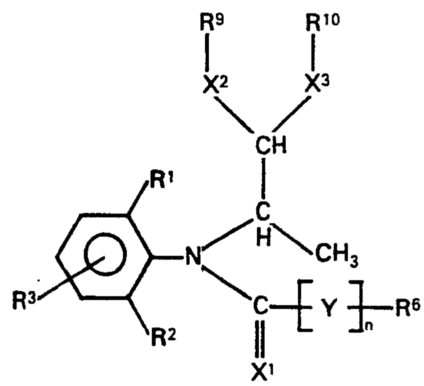

where $R^1$, $R^2$, $R^3$, $R^6$, $X^1$, $X^2$, $X^3$, Y and n have the above meanings and $R^9$ and $R^{10}$ independently of one another denote substituted or unsubstituted alkyl or arylalkyl, is transacetalized with a 1,2- or 1,3-diol, -dithiol or -mercapto-alcohol to give a cyclic acetal, at from 0 to 120°C, in the presence or absence of a solvent or diluent and with or without addition of a reaction accelerator.

9. A 2-(N-aryl-N-acyl)-aminopropanal-acetal as claimed in claim 1, selected from the group consisting of 2-(N-2′,6′-dimethylphenyl-, N-methoxyacetyl)-aminopropanal-dimethylacetal, 2-(N-2′,6′-dimethylphenyl, N-2″-furan-carbonyl)-amino-propanal-dimethylacetal, 2-methoxyacetic acid-N-(1-(1,3-dioxolan-2-yl)-ethyl)-2′6′-dimethylanilide, furan-2-carboxylic acid-N-(1-(1,3-dioxolan-2-yl)-ethyl)-2′,6′-dimethylanilide and 2-methoxyacetic acid-N-(1-(5-methyl-5-phenyl-1,3-dioxan-2-yl)-ethyl)-2′,6′-dimethylanilide.

10. A fungicide as claimed in claim 2, containing a 2-(N-aryl-N-acyl)-aminopropanal-acetal selected from the group consisting of 2-(N-2′,6′-dimethylphenyl-, N-methoxyacetyl)-amino-propanal-dimethylacetal, 2-(N-2′,6′-dimethylphenyl-, N-2′′-furan carbonyl)-amino-propanal dimethylacetal, 2-methoxy-acetic acid-N-(1-(1,3-dioxolan-2-yl)-ethyl)-2′,6′-dimethyl-anlide, furan-2-carboxylic acid-N-(1-(1,3-dioxolan-2-yl)-ethyl)-2′,6′-dimethylanilide and 2-methoxyacetic acide-N-(1-(5-methyl-5-phenyl-1,3-dioxan-2-yl)-ethyl-2′,6′-dimethyl-anilide.

**Revendications**
pour les Etats contractants: BE, CH, LI, DE, FR, GB, IT, LU, NL, SE

1. 2-(N-aryl-, N-acyl)-aminopropanalacétals de formule générale I

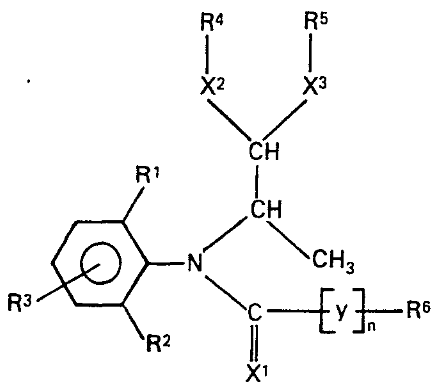

dans laquelle
$X^1$, $X^2$ et $X^3$ représentent indépendamment l'un de l'autre oxygène ou soufre,
Y oxygène, soufre ou $NR^7$,
n 0 ou 1,
$R^1$ alkyle en $C_1$ à $C_4$,
$R^2$ hydrogène, halogène, alkyle en $C_1$-$C_4$ ou alcoxy en $C_1$-$C_4$,
$R^3$ hydrogène, halogène ou alkyle en $C_1$-$C_4$,
$R^4$ et $R^5$, indépendamment l'un de l'autre, des restes alkyle ou aryllalkyle, éventuellement substitués, ou ensemble en tant que la partie groupe alkylène d'un noyau hétérocyclique à 5 ou 6 chaînons, éventuellement substitué par alkyle ou aryle,
$R^6$ un reste alkyle, alcényle, cycloalkyle, cycloalcényle ou hétéroaryle, éventuellement substitué, à l'exclusion du reste chlorométhyle et du reste acétylméthyle et, pour le cas où n=1, $R^6$ représente, en plus des significations données ci-dessus, un reste alcinyle, aryle, arylalkyle, ou hétéroarylalkyle éventuellement substitué et
$R^7$ représente hydrogène, un groupe alkyle ou alcoxy.

2. Fongicide contenant un 2-(N-aryl-, N-acyl)-aminopropanalacétal selon la revendication 1.

3. Fongicide contenant unsupport solide ou liquide et un 2-(N-aryl-, N-acyl)-aminopropanalacétal selon la revendication 1.

4. Procédé de préparation d'unfongicide, caractérisé par le fait qu'on mélange un support solide ou liquide avec un 2-(N-aryl-, N-acyl)-aminopropanalacétal selon la revendication 1.

5. Procédé de lutte contre les champignons, caractérisé par le fait qu'on traite les champignons ou les objets à protéger contre les champignons avec un 2-(N-aryl-, N-acyl)-aminopropanalacétal selon la revendication 1.

6. Procédé de préparation d'un 2-(N-aryl-, N- acyl)-aminopropanalacétal selon la revendication 1, caractérisé par le fait qu'à des températures comprises entre 0 et 120°C, éventuellement en présence d'un solvant ou diluant, éventuellement avec addition d'une base inorganique ou organique et éventuellement avec addtion d'un accélérateur de réaction, on fait agir une aniline de formule

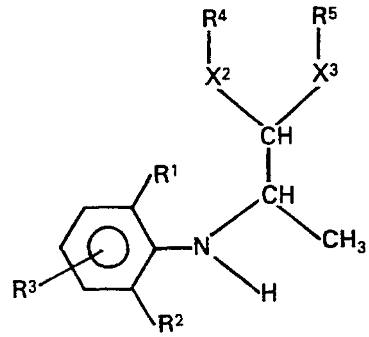

dans laquelle $R^1$, $R^2$, $R^3$, $R^4$, $R^5$, $X^2$ et $X^3$ ont les significations indiquées plus haut,

a) avec un halogénure d'acide de formule

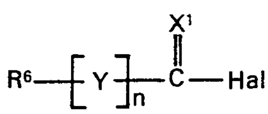

ou

b) avec un anhydride d'acide de formule

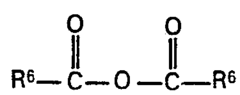

ou

c) avec un isocyanate de formule

$R^6—N=C=X^1$

dans lesquelles $X^1$, Y, n et $R^6$ ont les significations indiquées plus haut.

7. Procédé de préparation d'un 2-(N-aryl-, N-acyl)-amino-propanalacétal selon la revendication 1, caractérisé par le fait qu'à des températures comprises entre -20 et +100°C, éventuellement en présence d'un solvant ou diluant, éventuellement en présence d'une base inorganique ou organique et éventuellement avec addition d'un accélérateur de réaction, on fait réagir une aniline de formule:

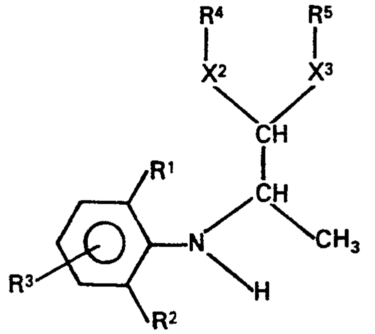

dans laquelle $R^1$, $R^2$, $R^3$, $R^4$, $R^5$, $X^2$ et $X^3$ ont les significations indiquées plus haut, d'abord;

a) avec un phosgène de formule

$X^1 = CCl_2$

dans laquelle $X^1$ a la signification indiquée plus haut, pour obtenir un composé de formule

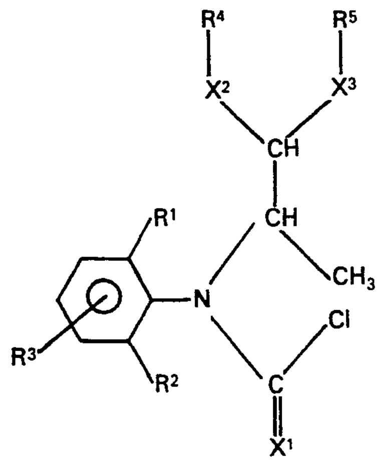

ou

b) avec un composé de formule:

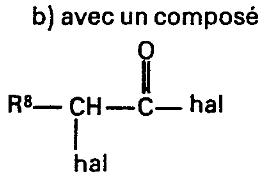

dans laquelle $R^8$ représente l'hydrogène ou a les significations données pour $R^6$ et hal représente un halogène, pour obtenir un composé de formule

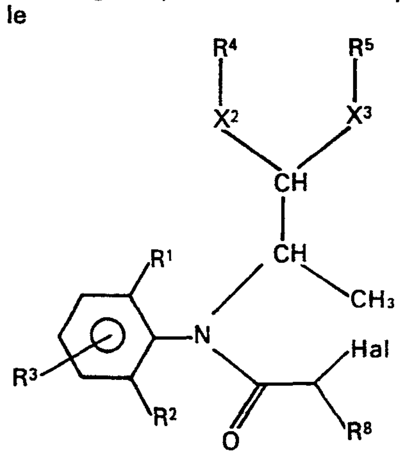

puis on fait réagir les composés indiqués ci-dessus avec des composés nucléophiles de formule

$R^6$-YH

dans laquelle Y et $R^6$ ont les significations données plus haut.

8. Procédé de préparation d'un 2-(N-aryl-, N-acyl)-aminopropanalacétal selon la revendication 1, caractérisé par le fait qu'à des températures comprises entre 0 et 120'C, éventuellement en présence d'un solvant ou diluant et éventuellement avec addition d'un accélérateur de réaction, on transforme en acétals cycliques par transacétalisation de composés de formule

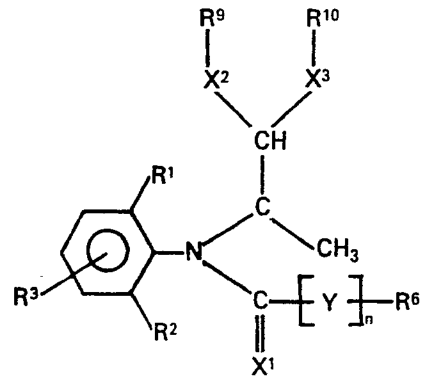

dans laquelle $R^1$, $R^2$, $R^3$, $R^6$, $X^1$, $X^2$, $X^3$, Y et n ont les significations ci-dessus et $R^9$ et $R^{10}$ représentent, indépendamment l'un de l'autre, des restes alkyle ou arylalkyle, éventuellement substitués.

9. 2-(N-aryl-, N-acyl)-aminopropanalacétal selon la revendication 1, choisi dans le groupe constitué de 2-(N-2',6'-diméthylphényl-, N-méthoxyacétyl)-aminopropanaldiméthylacétal, 2-(N-2',6'-diméthylphényl-, N-furan-2''-carbonyl,)-amino-propanaldiméthylacetal, N-(1-(1,3-dioxolan-2-yl)-éthyl-2',6'-diméthyl-anilide d'acide 2-méthoxy-acétique, N-(1-(1,3-dioxolan-2-yl)-éthyl)-2',6'-diméthylanilide d'acide furane-2-carboxylique et N-(1-(5-méthyl-5-phényl-1,3-dioxan-2-yl)-éthyl)-2',6'-diméthylanilide d'acide 2-méthoxyacétique.

10. Fongicide selon la revendication 2, contenant un 2-(N-aryl-, N-acyl)-aminopropanalacétal choisi dans le groupe constitué de 2-(N-2',6'-diméthylphényl-, N-méthoxyacétal)-aminopropanaldiméthylacétal, 2-(N-2',6'-dimetéhylphényl-, N-furan-2''-carbonyl)-aminopropanaldiméthylacétal, N-(1-(1,3-dioxolan-2-yl)-éthyl-2',6'-diméthylanilide d'acide 2-méthoxy-acétique, N-(1-(1,3-dioxolan-2-yl)-éthyl)-2',6'-diméthylanilide d'acide furane-2-carboxylique et N-(1-(5-méthyl-5- -phényl-1,3-dioxan-2-yl)-éthyl)-2',6'-diméthylanilide d'acide 2-méthoxyacétique.

**Claims**
for the Contracting State: AT

1. A fungicide containing a 2-(N-aryl-n-acyl)-aminopropanalacetal of the formula

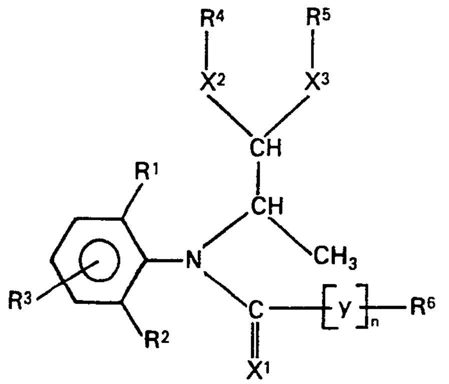

where
$X^1$, $X^2$, and $X^2$ independently of one another are oxygen or sulfur,
Y is oxygen, sulfur or $NR^7$,
n is 0 or 1,
$R^1$ is $C_1$-$C_4$-alkyl,
$R^2$ is hydrogen, halogen, $C_1$-$C_4$-alkyl or $C_1$-$C_4$-alkoxy,
$R^3$ is hydrogen, halogen or $C_1$-$C_4$-alkyl,
$R^4$ and $R^5$ independently of one another are unsubstituted or substituted alkyl or arylalkyl or together are alkylene and form part of a 5-membered or 6-membered heterocyclic ring which may or may not be substituted by alkyl or aryl,
$R^6$ is unsubstituted or substituted alkyl, alkenyl, cycloalkyl, cycloalkenyl or heteroaryl, with the exception of chloromethyl and acetylmethyl, and if n is 1, $R^6$ may also be unsubstituted or substituted alkynyl, aryl, arylalkyl or heteroarylalkyl, and
$R^7$ is hydrogen, alkyl or alkoxy.

2. A fungicide containing a solid or liquid carrier and a 2-(N-aryl-N-acyl)-aminopropanal-acetal as defined in claim 1.

3. A process for manufacturing a fungicide, characterized in that a solid or liquid carrier is mixed with a 2-(N-aryl-N-acyl)-aminopropanal-acetal as defined in claim 1.

4. A process for combating fungi, characterized in that the fungi of the objects to be protected against fungus attack are treated with a 2-(N-aryl-N-acyl)-aminopropanal-acetal as defined in claim 1.

5. A process for the manufacture of a 2-(N-aryl-N-acyl)-aminopropanal-acetal as defined in claim 1, characterized in that an aniline of the formula

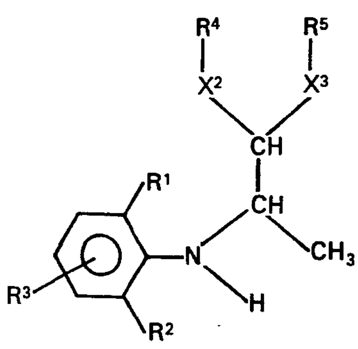

where $R^1$, $R^2$, $R^3$, $R^4$, $R^5$, $X^2$ and $X^3$ have the above meanings, is reacted

a) with an acid halide of the formula

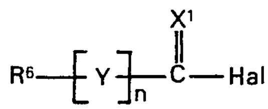

or

b) with an acid anhydride of the formula

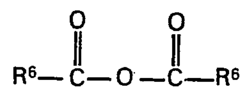

or

c) with an isocyanate of the formula

$R^6$-N=C=$X^1$

where $X^1$, Y, n and $R^6$ have the above meanings, in the presence or absence of a solvent or diluent, with or without addition of an inorganic or organic base, and with or without addition of a reaction accelerator, at from 0 to 120°C.

6. A process for the manufacture of a 2-(N-aryl-N-acyl)-aminopropanal-acetal as defined in claim 1, characterized in that an aniline of the formula

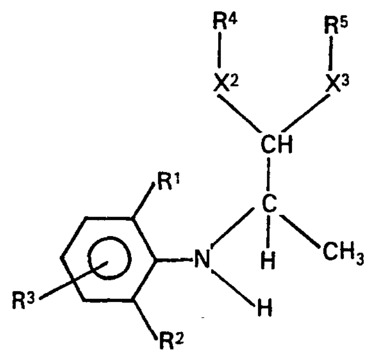

where $R^1$, $R^2$, $R^3$, $R^4$, $R^5$, $X^2$ and $X^3$ have the above meanings, is first reacted

a) with a phosgene of the formula VI

$X^1$=$CCl_2$,

where $X^1$ has the above meanings, to give a compound of the formula

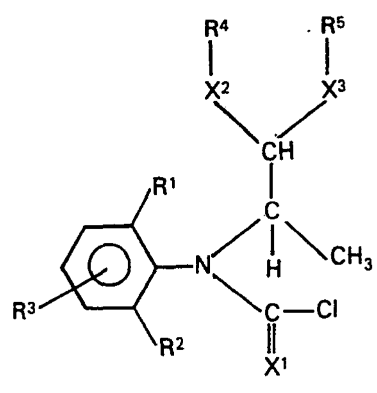

or

b) with a compound of the formula

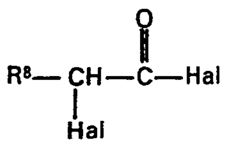

where $R^8$ is hydrogen or has the meanings given for $R^6$ and Hal is halogen, to give a compound of the formula

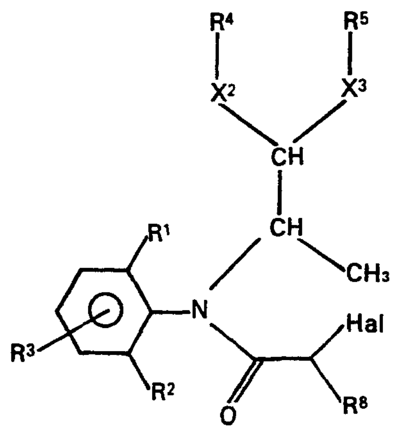

after which the above compounds of the formulae are reacted with a nucleophilic compound of the formula

$R^6$-YH,

where Y and $R^6$ have the above meanings, in the presence or absence of a solvent or diluent, in the presence of absence of an inorganic or organic base, and with or without addition of a reaction accelerator, at from -20 to +100°C.

7. A process for the manufacture of a 1-(N-aryl-N-acyl)-aminopropanal-acetal as defined in claim 1, characterized in that a compound of the formula

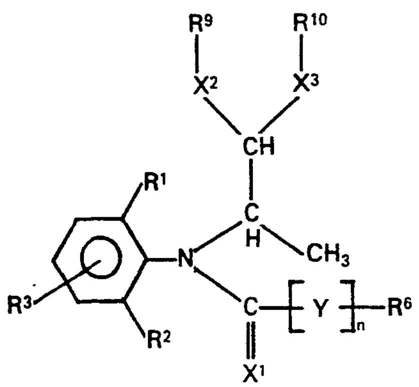

where $R^1$, $R^2$, $R^3$, $R^6$, $X^1$, $X^2$, $X^3$, Y and n have the above meanings and $R^9$ and $R^{10}$ independently of one another denote substituted or unsubstituted alkyl or arylalkyl, is transacetalized with a 1,2- or 1,3-diol, -dithiol or -mercapto-alcohol to give a cyclic acetal, at from 0 to 120°C, in the presence or absence of a solvent or diluent and with or without addition of a reaction accelerator.

8. A fungicide as claimed in claim 1, containing a 2-(N-aryl-N-acyl)-aminopropanal-acetal selected from the group consisting of 2-(N-2′,6′-dimethylphenyl-, N-methoxy-acetyl)-aminopropanal-dimethylacetal, 2-(N-2′,6′-dimethyl-phenyl-, N-2″-furan-carbonyl)-aminopropanal-dimethylacetal, 2-methoxyacetic acid-N-(1-(1,3-dioxolan-2-yl)-ethyl)-2′,6′-dimethylanilide, furan-2-carboxylic acid-N-(1-(1,3-dioxolan-2-yl)-ethyl)-2′,6′-dimethylanilide and 2-methoxyacetic acid-N-(1-(5-methyl-5-phenyl-1,3-dioxan-2-yl)-ethyl)-2′,6′-dimethyl-anilide.

**Revendications**

pour l'Etat contractant: AT

1. Fongicide contenant un 2-(N-aryl-N-acyl)-aminopropanalacétal de formule générale I

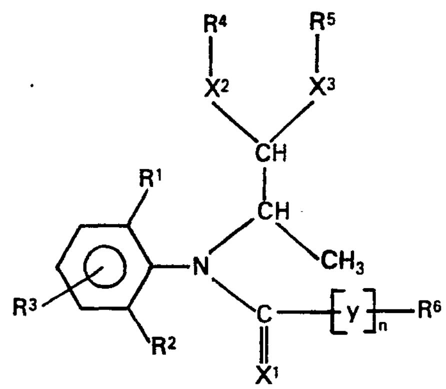

dans laquelle

$X^1$, $X^2$ et $X^3$ représentent indépendamment l'un de l'autre oxygène ou soufre,

Y oxygène, soufre ou $NR^7$,

n 0 ou 1,

$R^1$ alkyle en $C_1$ à $C_4$,

$R^2$ hydrogène, halogène, alkyle en $C_1$-$C_4$ ou alcoxy en $C_1$-$C_4$,

$R^3$ hydrogène, halogène ou alkyle en $C_1$-$C_4$,

$R^4$ et $R^5$, indépendamment l'un de l'autre, des restes alkyle ou arylalkyle, éventuellement substitués, ou ensemble en tant que la partie groupe alkylène d'un noyau hétérocyclique à 5 ou 6 chaînons, éventuellement substitué par alkyle ou aryle,

$R^6$ un reste alkyle, alcényle, cycloalkyle, cycloalcényle ou hétéroaryle, éventuellement substitué, à l'exclusion du reste chlorométhyle et du reste acétylméthyle et, pour le cas où n = 1, $R^6$ représente, en plus des significations données ci-dessus, un reste alcinyle, aryle, arylalkyle ou hétéroarylalkyle éventuellement substitué et

$R^7$ représente hydrogène, un groupe alkyle ou alcoxy.

2. Fongicide contenant un support solide ou liquide et un 2-(N-aryl-, N-acyl)-aminopropanal-acétal tel que défini dans la revendication 1.

3. Procédé de préparation d'un fongicide, caractérisé par le fait qu'on mélange un support solide ou liquide avec un 2-(N-aryl-, N-acyl)-aminopropanalacétal tel que défini dans la revendication 1.

4. Prodédé de lutte contre les champignons, caractérisé par le fait qu'on traite les champignons ou les objets à protéger contre les champignons avec un 2-(N-aryl-, N-acyl)-aminopropanalacétal tel que défini dans la revendication 1.

5. Procédé de préparation d'un 2-(N-aryl-N-acyl)-aminopropanalacétal tel que défini dans la revendication 1, caractérisé par le fait qu'à des températures comprises entre 0° et 120°C, éventuellement en présence d'un solvant ou diluant, éventuellement avec addition d'une base inorganique ou organique et éventuellement avec addition d'un accélérateur de réaction, on fait agir une aniline de formule

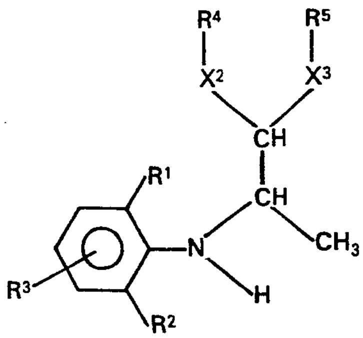

dans laquelle $R^1$, $R^2$, $R^3$, $R^4$, $R^5$, $X^2$ et $X^3$ ont les significations indiquées plus haut,

a) avec un halogénure d'acide de formule

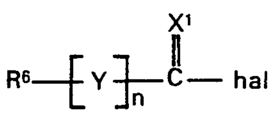

ou

b) avec un anhydride d'acide de formule

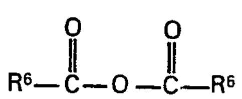

ou

c) avec un isocyanate de formule

$R^6—N=C=X^1$

dans lesquelles $X^1$, Y, n et $R^6$ ont les significations indiquées plus haut.

6. Procédé de préparation d'un 2-(N-aryl-, N-acyl)-aminopropanalacétal tel que défini dans la revendication 1, caractérisé par le fait qu'à des températures comprises entre -20° et + 100°C éventuellement en présence d'un solvant ou diluant, éventuellement en présence d'une base inorganique ou organique et éventuellement avec addition d'un accélérateur de réaction, on fait réagir une aniline de formule

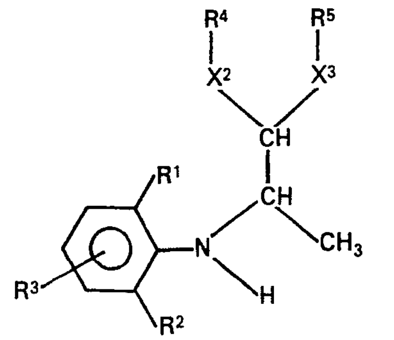

dans laquelle $R^1$, $R^2$, $R^3$, $R^4$, $R^5$, $X^2$ et $X^3$ ont les significations indiquées plus haut, d'abord

a) avec un phosgène de formule

$X^1 = CC1_2$

dans laquelle $X^1$ a la signification donnée plus haut, pour obtenir un composé de formule

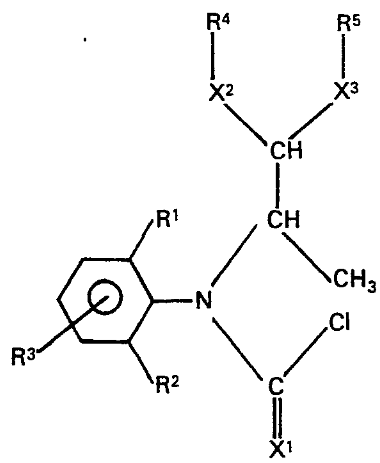

ou

b) avec un composé de formule

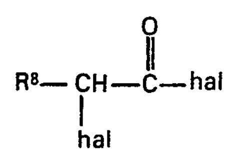

dans laquelle $R^8$ représente l'hydrogène ou a les significations données pour $R^6$ et hal représente un halogène, pour obtenir un composé de formule

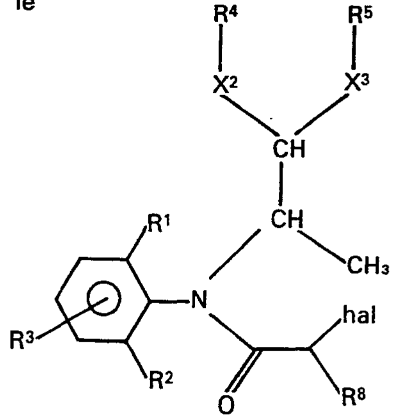

puis on fait réagir les composés indiqués ci-dessus avec des composés nucléophiles de formule

$R^6$ - YH

dans laquelle Y et $R^6$ ont les significations données plus haut.

7. Procédé de préparation d'un 2-(N-aryl-, N-acyl)-aminopropanalacétal tel que défini dans la revendication 1, caractérisé par le fait qu'à des températures comprises entre 0 et 120°C, éventuellement en présence d'un solvant ou diluant et éventuellement avec addition d'un accélérateur de réaction, on transforme en acétals cycliques par transacétalisation de composés de formule

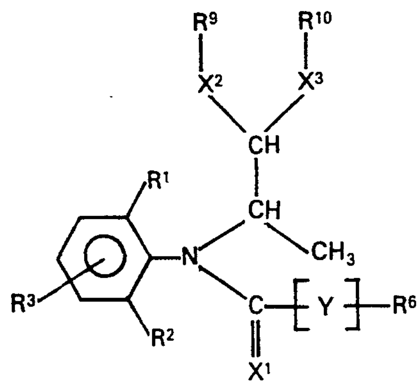

dans laquelle $R^1$, $R^2$, $R^3$, $X^1$, $X^2$, $X^3$, Y et n ont les significations ci-dessus et $R^6$ et $R^{10}$ représentent, indépendamment l'un de l'autre, des restes alkyle ou arylalkyle éventuellement substitués.

8. Fongicide selon la revendication 1, contenant un 2-(N-aryl-, N-acyl)-aminopropanalacétal choisi dans le groupe constitué de 2-(N-2',6'-diméthylphényl-, N-méthoxyacétyl)-aminopropanaldiméthylacétal, 2-(N-2',6'-diméthylphényl-,N-furan-2''-carbonyl)-aminopropanaldiméthylacétal, N-(1-(1,3-dioxolan-2-yl)-éthyl)-2',6'-diméthylanilide d'acide 2-méthoxy-acétique, N-(1-(1,3-dioxolan-2-yl)-2',6'-diméthylanilide d'acide furane-2-carboxylique et N-(1-(5-méthyl-5- -phényl-1,3-dioxan-2-yl)-éthyl)-2',6'-diméthylanilide d'acide 2-méthoxyacétique.